# FASCICULE DE BREVET EUROPEEN

(11) **EP 0 669 991 B1**
(45) Date de publication et mention de la délivrance du brevet: **03.11.1999**
(21) Numéro de dépôt: 94928418.6
(22) Date de dépôt: 15.09.1994
(51) Int. Cl.: C12Q 1/68, C08G 81/02

(54) **REACTIF ET PROCEDE POUR LA DETECTION D'UNE SEQUENCE NUCLEOTIDIQUE AVEC AMPLIFICATION DE SIGNAL**
REAGENS UND VERFAHREN FÜR DEN NACHWEIS EINER NUKLEOTIDISCHEN SEQUENZ MIT SIGNALAMPLIFIZIERUNG
REAGENT AND METHOD FOR THE DETECTION OF A NUCLEOTIDE SEQUENCE WITH SIGNAL AMPLIFICATION

(30) Priorité: 15.09.1993 FR 9311006
(43) Date de publication de la demande: 06.09.1995
(73) Titulaire: BIO MERIEUX, 69280 Marcy l'Etoile (FR)
(72) Inventeur: MANDRAND, Bernard, F-69100 Villeurbanne (FR); CROS, Philippe, F-69005 Lyon (FR); DELAIR, Thierry, F-69007 Lyon (FR); CHARLES, Marie-Hélène, F-69420 Condrieu (FR); EROUT, Marie-No[lle, F-69110 Sainte-Foy-les-Lyon (FR); PICHOT, Christian, F-69960 Corbas (FR)
(74) Mandataire: Tonnellier, Jean-Claude
(86) Numéro de dépôt international: FR9401084
(87) Numéro de publication internationale: WO9508000

(56) Documents cités:
- EP-A- 0 317 077
- EP-A- 0 450 594
- WO-A-91/08307
- US-A- 4 925 785

## Description

La présente invention a pour objet un réactif et procédé pour la détection d'une séquence nucléotidique dans un échantillon.

Il est souvent nécessaire, dans les technologies relatives aux acides nucléiques et au matériel génétique, de déterminer si un gène, une partie de gène ou une séquence nucléotidique est présente chez un organisme vivant, un extrait cellulaire de cet organisme ou un échantillon biologique. Puisque n'importe quel gène ou partie de gène est, en théorie, une séquence spécifique de bases nucléotidiques formant tout ou partie d'une molécule nucléotidique, il est possible de rechercher directement la présence d'une séquence nucléotidique spécifique au sein d'un échantillon.

L'intérêt de la recherche de séquences nucléotidiques spécifiques est immense pour la détection d'organismes pathogènes, la détermination de la présence d'allèles, la détection de la présence de lésions dans un génome hôte et la détection de la présence d'un ARNm particulier ou de la modification d'un hôte cellulaire, pour donner quelques exemples illustratifs. Les maladies génétiques telles que la maladie de Huntington, la myopathie de Duchenne, la phénylcétonurie et la béta-thalassémie peuvent être diagnostiquées par le biais de l'analyse de l'ADN des individus. De plus, le diagnostic ou l'identification des virus, viroïdes, bactéries, champignons, parasites peut être réalisé par des expériences d'hybridation avec des sondes nucléiques.

Différents types de méthodes de détection des acides nucléiques sont décrits dans la littérature. Ces méthodes reposent sur les propriétés d'appariement purine-pyrimidine des brins complémentaires d'acides nucléiques dans les duplex ADN-ADN, ADN-ARN et ARN-ARN. Ce processus d'appariement s'effectue par l'établissement de liaisons hydrogène entre les bases adénosine-thymine (A-T) et guanosine-cytosine (G-C) de l'ADN double brin. Des paires de bases adénosine-uracile (A-U) peuvent également se former par liaisons hydrogène dans les duplex ADN-ARN ou ARN-ARN. L'appariement de brins d'acides nucléiques pour la détermination de la présence ou de l'absence d'une molécule d'acide nucléique donnée est communément appelée par "hybridation d'acides nucléiques" ou simplement "hybridation".

Sur la base des propriétés des acides nucléiques, des techniques ont été développées permettant de mettre en évidence et de quantifier, dans un échantillon à analyser, un acide nucléique appelé cible. Ces techniques peuvent être divisées en deux grands groupes: les techniques dites de détection directe telles que celles développées par SOUTHERN. E. M. (J. Mol. Biol., 98, 503, (1975)) et la technique dite "Dot-blot" (MANIATIS et al., Molecular Cloning, Cold Spring Harbor, (1982)) pour la détection d'ADN ou la technique NORTHERN pour la détection d'ARN et les techniques dites indirectes telles que la technique sandwich ou "Reverse Dot" (voir par exemple DUNN A.R. et HASSEL J.A, Cell, **12**, 23 (1977)); RANKI M. et al., Gene, **21**, 77 (1983); PALVA A. et al., FEMS Microbiol. Lett. **23**, 83 (1984) ; POLSKY-CYNKI R. et al., Clin. Chem., **31**, 1438 (1985); Saiki R.K. et al, Proc. Natl. Acad. Sci.USA, vol 86, 6230-6234 (1989)).

Une des principales difficultés rencontrées lors de la mise au point d'un test pour détecter une séquence nucléotidique cible d'intérêt est le seuil de sensibilité des méthodes d'hybridation. En conséquence, diverses méthodes ont été décrites afin d'accroître la puissance de détection de ces techniques d'hybridation. Ces méthodes dites "d'amplification" peuvent intervenir à différents stades d'un procédé de détection par sondes nucléiques. Ces stades peuvent être classés en deux catégories: l'amplification de cible ou de signal. Les articles de Lewis (1992. Genetic Engineering News, 12, 1-9) d'une part, et d'Abramson et Myers (1993. Curr. Opin. Biotechnol., **4**, 41-47), d'autre part, constituent de bonnes revues générales de l'amplification de cible.

L'inconvénient majeur de ces techniques réside dans la difficulté à quantifier la cible nucléique d'intérêt après l'étape d'amplification.

D'autres approches concernant l'amplification de signal ont été décrites.

Palva A.M. et al. dans le brevet US 4 731 325 et Urdea M.S. et al. dans le brevet EP 0 225 807. multiplient les sondes de détection pouvant s'hybrider sur la cible. Dans beaucoup de cas (nécessité de différencier des espèces proches en bactériologie ou mise en évidence de maladies génétiques), il n'est pas possible d'utiliser cette technique car une seule séquence spécifique sur la cible est utilisable pour hybrider une sonde de détection.

Un certain nombre de techniques décrites consiste à augmenter le nombre des traceurs, c'est à.dire de molécules capables de générer un signal, directement ou indirectement, sur la sonde de détection. Le traceur peut notamment être une biotine, un fluorophore, une enzyme ou un groupement radioactif. La sonde de détection est greffée à un polymère, qui peut être de nature nucléotidique, sur lequel sont fixés, le plus souvent par covalence, un nombre de traceurs supérieur à deux (voir Ward D.C. et al. US 4 687.732, Bahl C. et al. EP 0 373 956, Ylikoski J. et al. WO 88/02784, Kwiatkowski M. et al. WO 90/00622, Segev D. et al. EP 0 292 128, Haralambidis J. et al. WO 89/03849, Rabbani E. et al. EP 0 173 339).

L'un des inconvénients de ces techniques réside dans la nécessité de réaliser un couplage contrôlé entre la sonde et le nombre de traceurs. Ce double couplage n'est pas aisé à maîtriser pour avoir un maximum de traceurs pour une sonde de détection. Des systèmes où le traceur est incorporé de manière contrôlée, par exemple au cours de la synthèse automatique d'oligodésoxyribonucléotides, ont été décrits (Pieles U. et al., Nucleic Acids Research, **18**, 4355-4360 (1990) ou Misiura K. et al., Nucleic Acids Research, **18**, 4345-4354 (1990)). Mais dans ce cas, le nombre de traceurs incorporés est faible du fait des limitations de la synthèse sur support solide. De plus, lorsque le nombre de traceurs augmente, la sonde de détection est facilement masquée par les traceurs et le rendement d'hybridation avec la cible chute.

Ces mêmes inconvénients se retrouvent dans le système décrit par Schneider R.J. et al. dans le brevet US 4 882 269. Une sonde dite primaire de nature nucléotidique comportant une queue de type polymère quelconque s'hybride sur la cible. Cette queue peut porter un traceur révélable par une sonde secondaire adaptée. Dans le cas où la queue est de nature nucléotidique, la sonde secondaire est de nature nucléotidique et peut s'hybrider avec la queue. Pour que le système fonctionne, il faut une queue nucléotidique de grande taille de séquence différente de la sonde primaire et il faut multiplier les sondes secondaires marquées. Ceci est réalisé en pratique par des techniques de biologie moléculaire où la sonde primaire est clonée dans un phage tel que M13 et les sondes secondaires sont complémentaires de différentes séquences du phage.

Un autre système proche est décrit par Chiswell D.J. dans le brevet EP 0 153 873. Chiswell présente un essai où une partie d'une sonde nucléique dite primaire s'hybride avec la cible. Cette sonde primaire comprend une deuxième partie sur laquelle peut s'hybrider une deuxième sonde multimarquée dite secondaire. Dans la réalisation pratique, ces sondes primaires sont fabriquées par des techniques de biologie moléculaire comme le clonage (par exemple clonage d'une séquence spécifique de la cible dans un fragment M13) qui sont peu adaptées à des processus de fabrication à grande échelle.

Collins M.L. (EP 0 204 510) décrit un procédé dans lequel la cible nucléique est mise en contact avec une première sonde appelée sonde réceptrice, une deuxième sonde appelée sonde amplificatrice et une troisième sonde, appelée sonde marquée, capable de s'hybrider avec la deuxième sonde amplificatrice. La sonde réceptrice qui s'hybride avec la cible possède une queue nucléotidique homopolymérique (par exemple polyA). La sonde amplificatrice contient une séquence complémentaire de la queue (par exemple polyT). La sonde de marquage contient une séquence nucléotidique capable de s'hybrider avec la sonde amplificatrice (par exemple polyA marquée). Cette combinaison de sondes constitue un empilement conduisant à une amplification de signal.

Un autre type d'empilement est également décrit par Segev D. dans le brevet EP 0 450 594.

Dans ces deux cas, les empilements qui se produisent dans le milieu d'hybridation ne sont pas contrôlés et conduisent à une mauvaise reproductibilité et donc à des problèmes de quantification. De plus la multiplication d'étapes successives d'hybridation génère des pertes qui entraînent un gain médiocre en amplification de signal.

On a maintenant trouvé un réactif pour la détection, y compris la détection quantitative (dosage) d'une séquence nucléotidique d'intérêt dans un échantillon susceptible de la contenir éliminant les inconvénients précités et permettant de contrôler efficacement et quantitativement l'amplification du signal.

L'invention a pour objet un nécessaire pour la détection d'une séquence d'une séquence nucléotidique d'intérêt, avec amplification de signal, comprenant au moins une sonde nucléotidique marquée avec un traceur, caractérisé par le fait qu'il contient, dans des conteneurs appropriés, un réactif comprenant essentiellement un copolymère à squelette linéaire portant des substituants latéraux, dont la chaîne est constituée par un premier type de motifs répétitifs et par au moins un autre type de motifs répétitifs, dans lequel au moins une partie des motifs du premier type portent un substituant latéral comprenant une unité nucléotidique, un tel substituant latéral étant absent sur les autres types de motifs, chacune desdites unités nucléotidiques, toutes identiques, comprenant, au moins, une séquence nucléotidique capable d'hybridation avec ladite séquence d'intérêt et une séquence nucléotidique capable d'hybridation avec ladite sonde,
ledit réactif contenant en moyenne plus de deux desdites unités nucléotidiques, en équivalents molaires, par mole de polymère.

La séquence d'intérêt est notamment une séquence nucléotidique simple brin, éventuellement obtenue par dénaturation préalable d'un hybride (ADN-ADN, ADN-ARN ou ARN-ARN) selon les techniques classiques (physiques, chimiques ou enzymatiques). Le système d'amplification de signal de l'invention peut également servir à la détection d'une séquence double brin, en opérant selon la technique de la triple hélice à laquelle il peut s'appliquer.

Le copolymère du réactif de l'invention a un squelette linéaire essentiellement carboné. Autrement dit, la chaîne polymère est formée essentiellement d'une suite de liaisons covalentes carbone-carbone. Les unités nucléotidiques sont présentes sous forme de substituants latéraux liés au squelette polymère, directement ou indirectement, par des liaisons covalentes. Le squelette polymère peut porter éventuellement d'autres substituants latéraux présents sur un monomère de départ, y compris des substituants latéraux susceptibles d'être impliqués dans la liaison avec l'unité nucléotidique mais n'ayant pas réagi avec le précurseur de celle-ci. Le copolymère est en particulier un bipolymère.

Les motifs du copolymère qui ne sont pas impliqués dans l'établissement d'une liaison avec l'unité nucléotidique servent notamment à espacer, dans le copolymère, les motifs portant les unités nucléotidiques, et peuvent aussi servir à moduler, de façon connue, les propriétés du copolymère, par exemple les propriétés de solubilité.

Selon un premier mode de réalisation, le réactif utilisé selon l'invention est caractérisé par le fait que, dans une même unité nucléotidique, lesdites séquences capables d'hybridation sont distinctes et non chevauchantes, et que ladite séquence nucléotidique capable d'hybridation avec la sonde est alors une séquence choisie arbitrairement.

Dans un système utilisant un tel réactif, la sonde marquée peut être utilisée comme sonde de détection universelle. En outre, un tel système convient plus particulièrement bien pour effectuer la détection en une seule étape, notamment sans lavage(s) intermédiaire(s).

Dans un autre mode de réalisation, le réactif utilisé selon l'invention est caractérisé par le fait que dans une même unité nucléotidique, l'une desdites séquences capables d'hybridation est incluse dans l'autre. Autrement dit, l'une desdites séquences est constituée par une partie de l'autre séquence, ou bien, à la limite, les deux séquences sont confondues (elles sont identiques) et, en fait, n'en font qu'une, dans l'unité nucléotidique. Dans ce mode de réalisation, la sonde marquée doit évidemment être adaptée à la cible (séquence d'intérêt) : ou bien la sonde est identique à la séquence d'intérêt, ou bien la sonde doit au moins être capable de s'hybrider avec la séquence complémentaire de la séquence d'intérêt.

Généralement, une molécule du réactif utilisé selon l'invention contient en moyenne de 3 à 100 et en particulier, de 5 à 50 unités nucléotidiques telles que définies ci-dessus.

Le réactif utilisé selon l'invention peut être considéré (indépendamment de son procédé réel d'obtention) comme un conjugué dans lequel les unités nucléotidiques sont couplées à un copolymère de base. Pour cette raison, on désigne parfois ce réactif, dans ce qui suit, comme un "conjugué de détection".

Le copolymère qui est à la base du réactif selon l'invention n'est pas nécessairement soluble dans les milieux aqueux (toujours utilisés en pratique dans les techniques de détection de cibles nucléiques), mais le conjugué de détection doit l'être.

Ce copolymère est de préférence un copolymère ayant une masse molaire comprise entre 5000 et 400000, par exemple entre 10000 et 150000.

Les unités nucléotidiques présentes dans le réactif d'amplification de l'invention sont généralement des oligonucléotides ayant une longueur de 5 à 100 nucléotides et de préférence de 10 à 40. En général, le réactif de l'invention contient, en équivalents molaires, de 3 à 100 unités nucléotidiques par mole de copolymère, de préférence de 5 à 50, par exemple de 10 à 30.

Selon des modes de réalisation particuliers de l'invention, pris isolément ou éventuellement en combinaison :
- le copolymère est un bipolymère ;
- ledit copolymère a un squelette linéaire essentiellement carbone ;
- lesdits motifs répétitifs proviennent de la copolymérisation d'un premier monomère insaturé avec au moins un autre monomère insaturé ;
- ledit premier monomère insaturé porte une fonction réactive capable de réagir (avec établissement d'une liaison covalente), soit directement, soit par l'intermédiaire d'un agent de couplage bifonctionnel, avec un précurseur de ladite unité nucléotidique pour former ledit substituant latéral ; la fonction réactive peut être protégée, de façon connue, pendant la copolymérisation, puis déprotégée pour la réaction avec le précurseur ;
- ladite fonction réactive est choisie parmi les fonctions amine, imide, aldéhyde, époxy, thiol, halogénoalkyle ou acide carboxylique, l'acide carboxylique étant éventuellement activé sous forme d'halogénure d'acide, sous forme d'anhydride ou sous forme d'ester.
- ledit premier monomère est un dérivé d'acide carboxylique insaturé ; notamment l'acide acrylique, méthacrylique ou maléique ; le dérivé est un anhydride, halogénure d'acide ou ester ;
- ledit autre monomère est choisi parmi la N-vinyl pyrrolidone, le méthylvinyléther, l'éthylène; le propylène et le styrène.

Le couplage d'unités nucléotidiques avec un copolymère peut être effectué selon les méthodes dites directes ou indirectes qui sont bien connues.

Par exemple, dans le cas d'une méthode directe, on synthétise un dérivé oligonucléotidique (précurseur de l'unité nucléotidique), ayant une fonction réactive à un endroit quelconque de la chaîne nucléotidique, comme par exemple à l'extrémité 5' ou à l'extrémité 3', ou sur une base nucléotidique ou sur un phosphate internucléotidique, ou encore sur la position 2' du sucre nucléotidique. Le dérivé oligonucléotidique est ensuite couplé à un copolymère préparé au préalable et comportant une fonction réactive complémentaire de celle du dérivé oligonucléotidique, c'est-à-dire telle que la réaction des deux fonctions réactives entre elles permet l'établissement d'une liaison covalente entre l'oligonucléotide et le copolymère. A titre d'exemples de couples de fonctions réactives, on peut coupler des amines primaires avec un acide carboxylique activé (par exemple sous forme d'anhydride ou d'ester de N-hydroxy succinimide) ou un aldéhyde, ou bien une fonction thiol avec un halogénoalkyle.

Dans la méthode de couplage indirecte, le polynucléotide et le copolymère sont chacun porteurs d'une fonction réactive, ces fonctions réactives pouvant être identiques ou différentes l'une de l'autre, ces deux fonctions n'étant pas complémentaires, mais étant capables de réagir avec un agent intermédiaire de couplage qui est un réactif bifonctionnel (homobifonctionnel si les deux fonctions sont identiques ou hétérobifonctionnel si les deux fonctions sont différentes). Parmi les agents de couplage homobifonctionnels, on peut citer le DITC (phénylène-1,4-diisothiocyanate), le DSS (disuccinimidylsubérate) ou analogues lorsque les deux fonctions réactives sont des fonctions amines primaires. Parmi les agents de couplage hétérobifonctionnels, on peut citer le SMCC (succinimidyl-4-(N-maléimidométhyl)cyclohexane-1-carboxylate) lorsque les deux fonctions réactives présentent chacune indépendamment de l'autre une fonction amine primaire et une fonction thiol, le SMPB (succinimidyl-4-(p-maléimido phényl) butyrate) ou analogues.

Après le couplage de l'oligonucléotide avec le copolymère, l'excès éventuel de fonctions réactives du copolymère est neutralisé de façon connue en soi. Par exemple, les groupements aldéhyde en excès peuvent être neutralisés par une amine primaire telle que l'éthanolamine, les groupements maléimide peuvent être neutralisés par un thiol (tel que la thioéthanolamine ou le dithiothréitol), etc.

On utilise en particulier des copolymères à base de N-vinylpyrrolidone (en abrégé NVP) ou d'anhydride maléique.

Le copolymère à base de NVP, de préférence un bipolymère, provient de la copolymérisation d'un monomère de N-vinylpyrrolidone et d'un deuxième monomère qui est un composé insaturé copolymérisable avec la NVP. Le deuxième monomère est notamment porteur d'une fonction réactive pour permettre l'établissement d'un couplage covalent entre les unités oligonucléotidiques et le copolymère. Par exemple, le deuxième monomère peut porter une fonction réactive telle que aldéhyde, époxy, halogénoalkyle, amine primaire, thiol, maléimide ou ester (de préférence une fonction ester de N-hydroxysuccinimide) ou une fonction activable telle qu'une fonction carboxyle (activable notamment par formation d'un ester d'hydroxysuccinimide) ou telle qu'une fonction hydroxyle (activable notamment par le bromure de cyanogène). En particulier, les deux monomères sont la N-vinylpyrrolidone et le N-acryloxysuccinimide (on obtient des copolymères appelés NVPNAS). Le copolymère comprend notamment de 25 à 70 %, en motifs, de motifs dérivés de la N-vinylpyrrolidone, les autres dérivant de l'acide acrylique.

Le copolymère à base d'anhydride maléique, de préférence un bipolymère, provient de la copolymérisation d'un monomère d'anhydride maléique et d'au moins un deuxième monomère, notamment un monomère insaturé, copolymérisable avec l'anhydride maléique. Le deuxième monomère est approprié pour permettre un comportement efficace du conjugué oligonucléotides-copolymère en solution, ce deuxième monomère ne présentant pas de fonction susceptible de réagir avec une unité oligonucléotidique. Le couplage covalent entre l'unité oligonucléotidique et le copolymère est effectué à l'aide de groupements anhydrides (provenant du monomère de départ) par réaction d'un dérivé de l'oligonucléotide précurseur de ladite unité oligonucléotide ou par l'intermédiaire d'un agent de couplage. Le deuxième monomère peut être l'éthylène, le styrène, le propylène, le méthylvinyléther. En particulier, les deux monomères sont l'anhydride maléique et le méthylvinyléther, et les copolymères correspondants sont appelés AMVE. Le copolymère comprend notamment de 35 à 65 %, en motifs, de motifs dérivés de l'anhydride maléique, en particulier entre 45 et 55 %.

Les copolymères anhydride maléique/méthylvinyléther (AMVE) et N-vinylpyrrolidone/N-acryloxysuccinimide (NVPNAS) sont en particulier des copolymères linéaires qui peuvent être obtenus généralement sous la forme de copolymères de type alterné.

L'invention concerne également l'utilisation d'un réactif, constitué par le conjugué de détection tel que défini précédemment, comme réactif d'amplification de signal dans un procédé de détection d'une séquence nucléotidique d'intérêt.

La séquence d'intérêt est soit une séquence de la substance cible (celle que l'on veut détecter effectivement), soit une séquence liée à la substance cible, soit encore (dans des méthodes de compétition) une séquence liée à un analogue de la substance-cible, compétiteur de celle-ci.

L'invention concerne notamment un procédé de détection, avec amplification de signal, d'une séquence nucléotidique d'intérêt susceptible d'être immobilisée sur un support solide, dans lequel on utilise une sonde nucléotidique de détection marquée avec un traceur, caractérisé par le fait que l'on ajoute à un milieu liquide en contact avec ledit support solide, dans des conditions permettant l'hybridation :
- un réactif tel que défini ci-dessus,
- et la sonde de détection marquée,
puis on révèle selon les méthodes usuelles la présence éventuelle du traceur immobilisé sur le support solide.

Le procédé de l'invention est applicable notamment à la détection et/ou au dosage d'un fragment d'acide nucléique cible susceptible d'être présent dans un échantillon. La séquence nucléotidique d'intérêt est une partie déterminée de la séquence nucléotidique de la cible, qui peut être choisie, notamment, comme moyen de caractérisation d'une espèce, d'un genre, d'un allèle ou analogues. La séquence nucléotidique d'intérêt est fixée sur le support solide directement ou indirectement par l'intermédiaire d'un ligand. Le ligand peut notamment être une sonde de capture choisie pour être complémentaire d'une autre région de la cible, selon la technique sandwich, qui est bien connue. Dans un autre mode de réalisation de la technique sandwich, la sonde de capture est elle-même greffée sur un copolymère pour être fixée sur le support solide par adsorption passive, c'est à dire sans formation d'une liaison covalente entre le support et le copolymère portant la sonde de capture. Cette méthode peut contribuer à réduire le bruit de fond. Par de simples expériences de routine, il est possible de choisir le ou les copolymères (identiques ou différents) permettant une bonne fixation de la sonde de capture, par adsorption passive, sur le support, et une amplification de signal accompagnée d'une réduction du bruit de fond, ce qui conduit à un gain en sensibilité. La séquence d'intérêt peut également être fixée directement sur le support solide par l'intermédiaire de la cible selon la technique "Reverse Dot".

Le procédé de l'invention est également applicable aux immunoessais et notamment à la détection d'haptènes, d'antigènes, de polypeptides ou d'anticorps dans des processus avec ou sans compétition. Par exemple, la séquence d'intérêt peut être greffée, par liaison covalente de préférence, à un ligand susceptible de réagir spécifiquement avec une molécule cible. Dans les techniques de compétition, la séquence d'intérêt peut être greffée, de préférence par covalence, à un ligand, ledit ligand étant susceptible d'entrer en compétition avec la cible pour sa fixation à un anti-ligand spécifique. Les techniques de couplage entre un ligand et la séquence d'intérêt dépendent de la nature du ligand et sont bien connues. Il est évidemment nécessaire de choisir une méthode de couplage qui n'altère pas la capacité du ligand à reconnaître l'anti-ligand, ce qui peut être facilement vérifié par de simples expériences de routine.

A titre illustratif, si la cible à doser est un antigène, la séquence d'intérêt est greffée sur un anticorps spécifique de l'antigène. Cet anticorps après réaction avec l'antigène peut réagir par l'intermédiaire de la séquence d'intérêt avec le réactif oligonucléotides-copolymère de l'invention. Une sonde de détection complémentaire d'au moins une partie de la séquence d'intérêt, greffée sur le copolymère, permet de révéler l'antigène avec une bonne sensibilité.

Si l'unité oligonucléotidique du réactif comprend une séquence spécifique de la séquence d'intérêt et une séquence arbitraire, la sonde de détection sera complémentaire de la séquence arbitraire. Une réduction du nombre d'étapes est possible en utilisant un tampon approprié permettant à la fois la réaction du ligand sur l'anti-ligand et l'hybridation des séquences nucléotidiques entre elles. Un essai en une étape est réalisable, l'anticorps étant greffé à la séquence d'intérêt, le réactif de détection copolymère-oligonucléotides et la sonde de détection réagissant pendant la même incubation.

De même, un deuxième conjugué oligonucléotides-copolymère est utilisable comme conjugué de capture dans un immunoessai selon la technique sandwich en combinaison avec le conjugué de détection, à condition que l'anti-ligand de capture soit couplé avec une séquence nucléotidique complémentaire, au moins en partie, de la séquence greffée sur le conjugué de capture. Une réduction du bruit de fond jointe à une augmentation de signal conduit, par un choix de conjugués appropriés, à une augmentation de la sensibilité.

Les copolymères utilisés selon l'invention sont de préférence des bipolymères. Ils peuvent être obtenus, de façon connue en soi, par voie radicalaire, par voie ionique, par réaction de polycondensation ou par réaction de transfert de groupe. De préférence, ce sont des copolymères obtenus par copolymérisation radicalaire de co-monomères à insaturation éthylénique dont l'un comporte une fonction réactive appropriée pour le greffage covalent des substituants latéraux nucléotidiques.

Le terme "support solide" tel qu'utilisé ici inclut tous les matériaux sur lesquels peut être immobilisé un oligonucléotide pour une utilisation dans des tests diagnostiques, en chromatographie d'affinité et dans des processus de séparation. Des matériaux naturels ou de synthèse, modifiés ou non chimiquement peuvent être utilisés comme support solide, notamment les polysaccharides tels que les matériaux à base de cellulose, par exemple du papier, des dérivés de cellulose tels que l'acétate de cellulose et la nitrocellulose, du dextran ; des polymères tels que polychlorures de vinyle, polyéthylènes, polystyrènes, polyacrylates, polyamides, ou copolymères à base de monomères vinyl aromatiques, alkylesters d'acides alpha-bêta insaturés, esters d'acides carboxyliques insaturés, chlorure de vinylidène, diènes ou composés présentant des fonctions nitrile (acrylonitrile); des polymères de chlorure de vinyle et de propylène ; des polymères de chlorure de vinyle et d'acétate de vinyle; des copolymères à base de styrènes ou dérivés substitués du styrène; des fibres naturelles telles que le coton et des fibres synthétiques telles que le nylon; des matériaux inorganiques tels que la silice, le verre, la céramique, le quartz; des latex c'est à dire une dispersion aqueuse colloïdale d'un polymère quelconque insoluble dans l'eau ; des particules magnétiques ; des dérivés métalliques. Le choix d'un matériau de support, dans chaque cas particulier, peut être effectué à partir de simples expériences de routine.

De préférence, le support solide utilisé dans la présente invention est un polymère de polystyrène, un copolymère butadiène-styrène ou un copolymère butadiène-styrène en mélange avec un ou plusieurs polymères ou copolymères choisis parmi le polystyrène, les copolymères styrène-acrylonitrile ou styrène-méthylméthacrylate de méthyle, les polypropylènes, les polycarbonates ou analogues. Le support solide utilisé est notamment un polystyrène ou un copolymère à base de styrène comprenant entre environ 10 et 90 % en poids de motifs de styrène.

Le support solide selon l'invention peut être, sous les formes usuelles appropriées, par exemple, sous la forme d'une plaque de microtitration, d'une feuille, d'un cône, d'un tube, d'un puits, de billes, de particules ou analogues.

Le terme "unité oligonucléotidique" tel qu'utilisé dans la présente demande, désigne un enchaînement d'au moins 5 désoxyribonucléotides ou ribonucléotides comprenant éventuellement au moins un nucléotide modifié, par exemple au moins un nucléotide comportant une base modifiée telle que l'inosine, la méthyl-5-désoxycytidine, la diméthylamino-5-désoxyuridine, la désoxyuridine, la diamino-2,6-purine, la bromo-5-désoxyuridine ou toute autre base modifiée permettant l'hybridation. Ce polynucléotide peut aussi être modifié au niveau de la liaison internucléotidique comme par exemple les phosphorothioates, les H-phosphonates, les alkyl-phosphonates, au niveau du squelette comme par exemple les alpha-oligonucléotides (FR 2 607 507) ou les PNA (M. Egholm et al., J. Am. Chem. Soc., (1992), **114,** 1895-1897). Ces diverses modifications peuvent éventuellement être prises en combinaison.

Les traceurs utilisés pour marquer la sonde de détection sont choisis parmi les traceurs usuels. Ce sont par exemple :
- des enzymes qui produisent un signal détectable par exemple par colorimétrie, fluorescence, luminescence, comme la peroxydase de raifort, la phosphatase alcaline, la bêta galactosidase, la glucose-6-phosphate déshydrogénase;
- des chromophores comme les composés fluorescents, luminescents, colorants ;
- des groupements à densité électronique détectable par microscopie électronique ou par leur propriété électrique comme la conductivité, l'ampérométrie, la voltamétrie, les mesures d'impédance ;
- des groupements détectables par des méthodes optiques (comme la diffraction, la résonance plasmon de surface, la variation d'angle de contact) ou par dès méthodes physiques comme la spectroscopie de force atomique, l'effet tunnel ;
- des molécules radioactives comme le ³² P, le ³⁵ S ou le ¹²⁵ I.

Les méthodes covalentes ou non covalentes pour attacher ces traceurs dépendent des traceurs et sont bien connues par l'homme de l'art.

Des systèmes indirects peuvent aussi être utilisés, par exemple, ceux comprenant les haptènes, détectables par un anticorps spécifique, ou une protéine comme le couple biotine/avidine ou streptavidine, ou encore un couple sucre/lectine. Dans ce cas c'est l'anticorps ou la protéine qui porte un traceur.

Les hybrides acides nucléiques de type ADN/ADN, ARN/ARN, ADN/ARN, peuvent être détectés par des anticorps antihybrides, ou par des protéines spécifiques comme les polymérases phagiques.

Si la sonde de détection est constituée de nucléotides modifiés comme les nucléotides d'anomérie alpha ou les PNA (P.E NIELSEN et al, Science, 254,1497-1500 (1991)), des anticorps anti-alphanucléotides ou anti-PNA peuvent être utilisés.

Le terme "anticorps" désigne notamment les anticorps monoclonaux ou polyclonaux, les fragments d'anticorps et les anticorps obtenus par recombinaison génétique.

Le terme "haptène" désigne une molécule de taille insuffisante pour être immunogène, mais qui par couplage avec une protéine, par exemple, permet par immunisation d'animaux l'obtention d'anticorps reconnaissant ladite molécule.

Par séquence homologue d'une autre séquence, on désigne une séquence capable de s'hybrider avec une séquence strictement complémentaire de ladite autre séquence.

On entend par fixation passive une fixation due à des forces autre que des forces de liaison covalente.

Dans les dessins annexés, où les mêmes lettres ou chiffres de référence désignent constamment le même élément :

La figure 1 décrit schématiquement le protocole sandwich direct selon les méthodes conventionnelles pour détecter un acide nucléique.
1 représente le support solide.
2 représente la sonde de capture fixée sur le support solide par un moyen quelconque.
3 représente la cible nucléique à détecter.
D représente la sonde de détection et T le traceur.

La figure 2 décrit un protocole sandwich avec utilisation d'un réactif conjugué de détection de l'invention pour amplifier le signal.
1 représente le support solide.
2 représente la sonde de capture fixée sur le support solide par un moyen quelconque.
3 représente la cible nucléique à détecter.
D représente la sonde de détection et T le traceur.

CJO représente le conjugué copolymère-oligonucléotides. L'unité oligonucléotidique (représentée par un rectangle noir) greffée sur le copolymère est complémentaire d'une partie de la cible dénommée séquence d'intérêt (représentée par un rectangle blanc). La sonde de détection D est homologue de la cible.

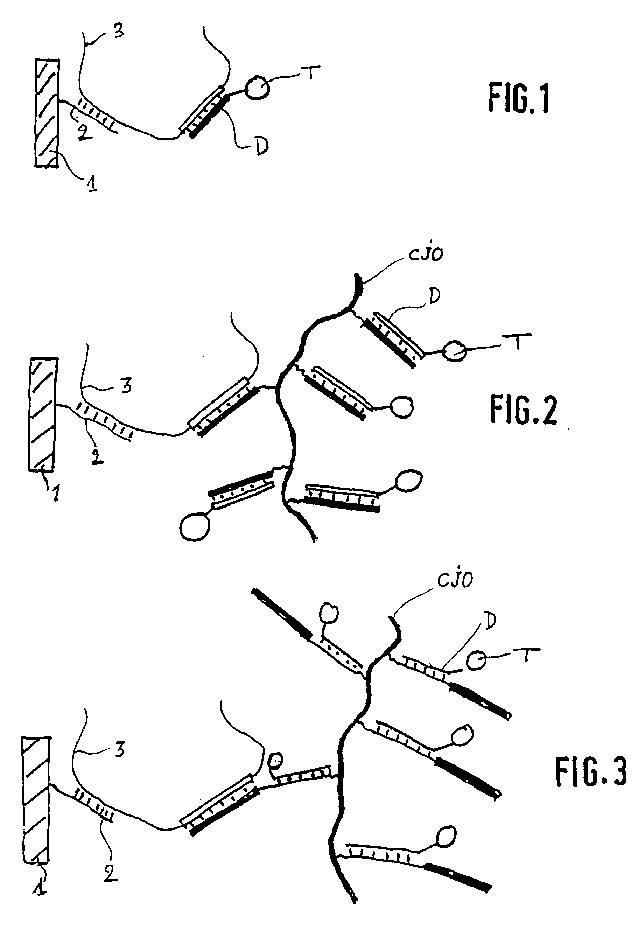

La figure 3 décrit un protocole sandwich avec utilisation d'un conjugué de détection pour amplifier le signal. Dans ce cas il ne peut y avoir compétition entre la cible et D.
1 représente le support solide.
2 représente la sonde de capture fixée sur le support solide par un moyen quelconque.
3 représente la cible nucléique à détecter.
D représente la sonde de détection et T le traceur.

CJO représente le conjugué copolymère-oligonucléotides. L'unité oligonucléotidique greffée sur le copolymère comprend 2 parties. L'une (représentée par un rectangle noir) est complémentaire de la séquence d'intérêt (représentée par un rectangle blanc). L'autre, représentée par un trait non épaissi, a une séquence arbitraire. La sonde de détection D est complémentaire de cette séquence arbitraire.

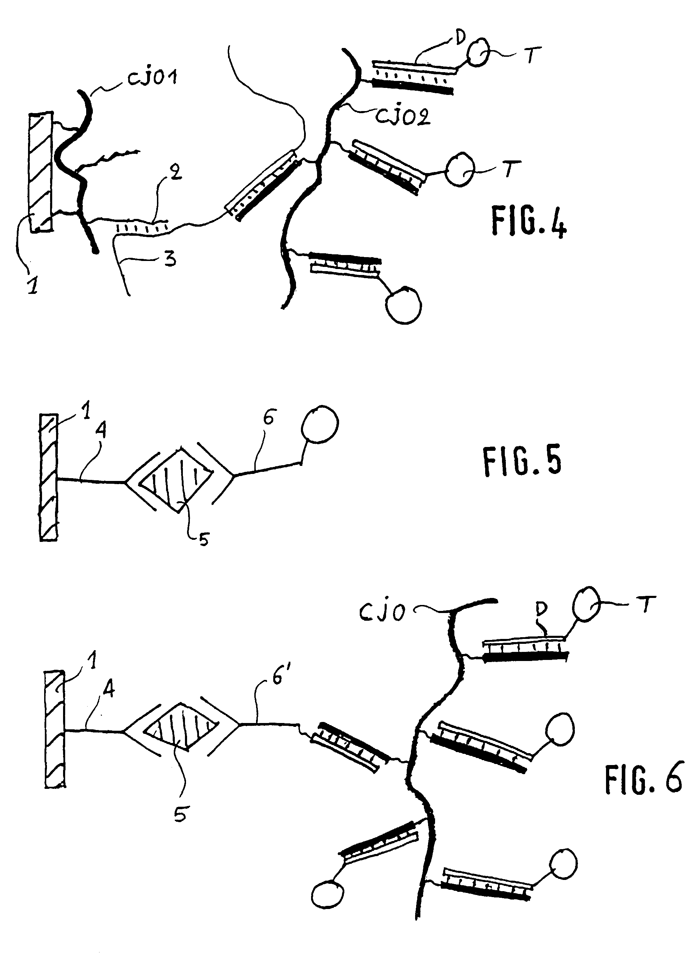

La figure 4 décrit un protocole sandwich avec utilisation d'un conjugué de détection et d'un conjugué de capture.
1 représente le support solide.
2 représente la sonde de capture fixée sur le support solide par l'intermédiaire d'un conjugué de capture CJO1. La sonde de capture est greffée de manière covalente sur le copolymère pour former CJO1 qui est fixé par adsorption passive sur le support.
3 représente la cible nucléique à détecter.
D représente la sonde de détection et T le traceur.

CJO2 représente le conjugué copolymère-oligonucléotides. L'unité oligonucléotidique (représentée par un rectangle noir) greffée sur le copolymère est complémentaire de la séquence d'intérêt (représentée par un rectangle blanc). La sonde de détection D est homologue de la séquence d'intérêt. Comme dans la figure 3, une unité oligonucléotidique comprenant une séquence arbitraire peut être utilisée. D est alors complémentaire de cette séquence arbitraire.

Les deux copolymères composant CJO1 et CJO2 peuvent être identiques ou différents mais les deux polynucléotides greffés respectivement sur ces copolymères sont complémentaires de deux régions distinctes de la cible à détecter.

La figure 5 décrit un protocole immunologique sandwich direct selon les méthodes conventionnelles pour la détection d'antigène.
1 représente le support solide.
4 représente un premier anticorps de capture fixé sur le support par un moyen quelconque.
5 représente un antigène.
6 représente un deuxième anticorps de détection couplé à un traceur.

La figure 6 décrit un protocole immunologique sandwich particulier utilisant un réactif conjugué de détection selon l'invention.
1 représente le support solide.
4 représente un premier anticorps de capture fixé sur le support solide par un moyen quelconque.
5 représente un antigène.
6' représente un deuxième anticorps de détection couplé de manière covalente à une séquence nucléotidique d'intérêt (représentée par un rectangle blanc).

CJO représente le conjugué copolymère-oligonucléotide. L'unité oligonucléotidique (représentée par un rectangle noir) greffée sur le copolymère est complémentaire de la séquence d'intérêt (représentée par un rectangle blanc) greffée sur le deuxième anticorps.

D représente la sonde de détection homologue de l'oligonucléotide du conjugué.

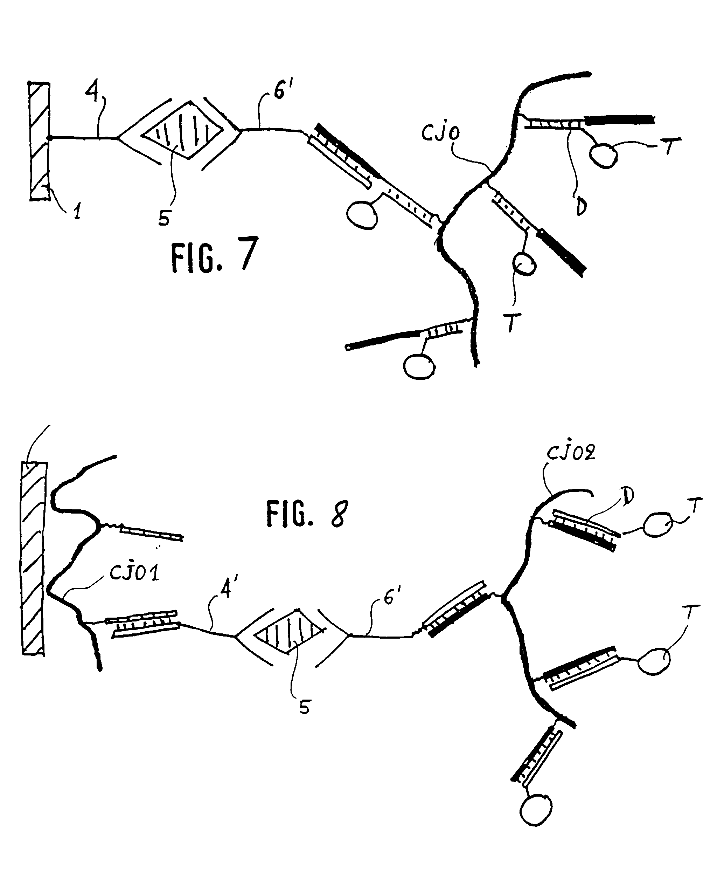

La figure 7 est l'équivalent pour la détection immunologique de la figure 3 pour les acides nucléiques.

La figure 8 est l'équivalent pour la détection immunologique de la figure 4 pour les acides nucléiques.

L'anticorps de capture est greffé sur un oligonucléotide identique ou non à la séquence d'intérêt couplée à l'anticorps 6', et forme le composé 4'.
4' est capturé sur la phase solide par un conjugué de capture CJO1.

### EXEMPLE 1 : Synthèse et caractérisation des copolymères :

Le protocole suivant constitue le mode opératoire général des réactions de copolymérisation.

Le tableau 1 résumera, par la suite, les conditions particulières de cette synthèse.
- Dans un ballon tricol de 100 ml purgé à l'azote, sont mélangés 50 ml de N-diméthylformamide anhydre (ALDRICH; référence 22705-6), une quantité x exprimée en gramme (g) de N-vinylpyrrolidone (ALDRICH; référence V 340-9) fraîchement distillée, ainsi qu'une quantité y (en gramme) de N-acryloxysuccinimide (KODAK ; référence 110 1690).
- Le milieu réactionnel porté à une température de 60°C, est agité pendant 2 heures, sous bullage régulier d'azote, afin d'éliminer toute trace d'oxygène.
- z g d'acide 4,4' Azobis (4-cyano pentanoïque) (FLUKA; référence 11 590) sont dissous dans 1 ml de N-N diméthyl-formamide anhydre. Ce composé constitue l'amorceur de la réaction de polymérisation radicalaire.
- Après un bullage d'azote pendant 15 minutes, la solution d'amorceur est ajouté rapidement au milieu réactionnel. Ceci constitue le temps zéro de la polymérisation.
- La réaction est conduite à 60°C, sous agitation, sous bullage lent et régulier d'azote.
- Des prélèvements sont effectués toutes les 3 minutes.

Pour stopper la réaction, on ajoute quelques grains d'un inhibiteur de polymérisation: l'hydroquinone (JANSSEN; référence 123-31-9). Les prélèvements sont ensuite placés dans la glace.
- Chacun de ces prélèvements est ensuite analysé en chromatographie en phase gazeuse (chromatographe DI 200, intégrateur ENICA 21, de la société DELSI INSTRUMENTS), grâce à une colonne SE 30 (méthylsilicone adsorbée sur Chromosorb 10 %). Ceci permet le suivi cinétique de la réaction de polymérisation c'est à dire la connaissance, à tout instant, de la quantité de monomères résiduels, donc du taux de conversion (relatif à chaque monomère et global).
   Conditions d'analyse :

   Pression Air = Pression H₂ = 1 bar (10⁵ Pa)
   Pression N₂ = 0,8 bar
   Température du four = 175°C
   Température de l'injecteur = Température du détecteur = 300°C
- Après un temps de réaction noté t et exprimé en heures, le milieu réactionnel est versé dans une ampoule à décanter, puis ajouté goutte à goutte dans un bécher contenant un grand excès d'éther éthylique (SDS, référence 440516) sous forte agitation.
- Le polymère, formé pendant la réaction, précipite sous la forme de cristaux blancs assez fins.

Quand tout le milieu réactionnel a été précipité, la solution d'éther éthylique est filtrée sur fritté n° 4. Le polymère est lavé à l'aide d'un grand excès d'éther éthylique puis séché pendant plusieurs heures dans une étude à vide, à température ambiante.
- Pour purifier le polymère (élimination des monomères résiduels ainsi que les produits de faible masse molaire), celui-ci est dissous dans le N-N diméthylformamide (DMF) puis reprécipité dans l'éther éthylique.
- Les copolymères ainsi obtenus sont conservés sous atmosphère inerte, à l'abri de l'humidité.

Le composé formé a la structure suivante :

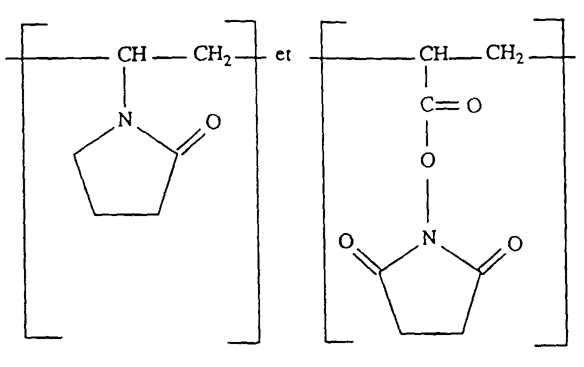

Le tableau 1 résume les conditions particulières à chaque réaction de polymérisation

**TABLEAU 1**

| Dénomination des Copolymères | x NVP grammes | y NAS grammes | C (*) moles/l | z AZCB gramme | t heures | R (**)% |
|---|---|---|---|---|---|---|
| COPO1 | 2,22 | 0,84 | 0,5 | 0,074 | 20 | 55 |
| COPO2 | 2,78 | 4,22 | 1,0 | 0,148 | 20 | 90 |
| COPO3 | 1,14 | 2,54 | 0,5 | 0,074 | 20 | 90 |
| COPO4 | 0,23 | 0,50 | 0,1 | 0,015 | 20 | 90 |
| COPO5 | 2,28 | 5,08 | 1,0 | 0,148 | 20 | 90 |

| | | | | | | |
|---|---|---|---|---|---|---|
| (*) C représente la concentration des monomères en moles/litre, en début de réaction. | | | | | | |
| (**) R représente la conversion, exprimée en pourcentage massique, des 2 monomères de départ. Abréviations utilisées dans le tableau 1 NVP = N-vinyl pyrrolidone NAS = N-acryloxysuccinimide AZCB = acide azobis 4-4' (4-cyanopentanoique) | | | | | | |

Les caractéristiques des copolymères utilisés dans la présente invention sont résumés dans le tableau 2 .

**TABLEAU 2**

| | Masse moléculaire (g / mole) (*) | % Molaire de NAS dans le copolymère final (**) |
|---|---|---|
| COPO1 | 60 000 (10%) | 39 |
| COPO2 | 160 000 (2%) | 57 |
| COPO3 | 70 000 (2%) | 62 |
| COPO4 | 19 500 (2%) | 59 |
| COPO5 | 160 000 (2 %) | 61 |

| | | |
|---|---|---|
| (*) Les masses moléculaires ont été déterminées par diffusion de la lumière (méthode statique). Solvant d'étude : DMF/eau = 90/10. Température : 20°C. Le chiffre entre parenthèses représente l'erreur de mesure sur la masse. | | |
| (**) Cette valeur a été déterminée par dosage des fonctions N-hydroxysuccinimide (NHS) en présence d'un excès de NH₄OH par spectroscopie UV à 260 nm avec un coefficient d'extinction molaire pour le NHS de 7100 1.mole ⁻¹.cm ⁻¹ dans un mélange eau / DMF / NH₄ OH (0.1M) = 80/10/10. | | |

L'erreur de mesure est de 5 %.

Le tableau 3 donne les déplacements chimiques par résonance magnétique nucléaire (RMN) des différents carbones du copolymère. Les carbones sont identifiés par le numéro tel que décrit dans le schéma ci-dessous :

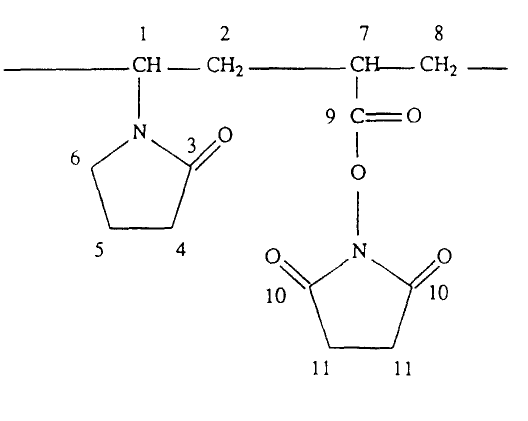

**TABLEAU 3**

| Position du carbone | Déplacement chimique |
|---|---|
| 1 | 48 (raie large) |
| 2 | 34 (raie large) |
| 3 | 175-177.5 (multiplet) |
| 4 | 32.2 (raie large) |
| 5 | 19 (singulet) |
| 6 | 42 (raie large) |
| 7 | 38.5 (raie large) |
| 8 | 34 (raie large) |
| 9 et 10 | 171 et 170.9 (doublet) |
| 11 | 26.4 et 26.65 (doublet) |
| Solvant : DMF deutéré Référence interne : Dioxanne avec une raie à 67,8 ppm. Les déplacements chimiques sont donnés en partie par million (ppm). | |

### Couplage d'unités oligonuléotidiques sur des copolymères :

Les polynucléotides utilisés dans la présente invention sont des oligodésoxyribonucléotides de synthèse préparés avec un synthétiseur automatique Applied Biosystems modèle 394 selon le protocole du fabricant.

Ces oligonucléotides possèdent à leur extrémité 5' un bras aliphatique NH₂ préparé selon la méthode décrite dans la demande de brevet WO 91/19812.

Par l'intermédiaire des fonctions esters du N-Hydroxysuccinimide, il y a réaction directe sur le bras aminé à l'extrémité 5' de l'oligonucléotide pour former une liaison amide.

Le protocole de couplage général est le suivant :
Une solution aqueuse contenant 32 nmoles d'oligonucléotides est versée dans un tube Eppendorf, puis évaporée dans un évaporateur à centrifugation sous vide.

Le résidu sec est repris dans 50 microlitres de tampon Borate 0,1 M pH = 9,3 puis l'on additionne 400 microlitres de DMF.

A cette solution sont ajoutés 50 microlitres d'une solution du copolymère à 1mg/ml dans le DMF (SDS, référence 340216).

Le tube Eppendorf contenant le milieu réactionnel est agité pendant 16 heures dans un agitateur chauffant pour tubes Eppendorf porté à une température de 37°C.

Le mélange (DMF/eau) est ensuite évaporé à l'évaporateur à centrifugation sous vide.

Le résidu sec est repris dans 50 microlitres d'eau distillée, afin d'être analysé par électrophorèse capillaire.

### Conditions expérimentales de la séparation par électrophorèse capillaire en solution libre :

- Appareil : 270 A-HT commercialisé par la société APPLIED-BIOSYSTEMS
- Capillaire de silice d'une longueur de 72 cm (APPLIED-BIOSYSTEMS, réf. 0602-0014)
- Electrolyte de séparation : Tampon Carbonate de Sodium 50mM, pH = 9,6
- Voltage appliqué : 20 kilovolts.
- Température : 40°C.
- Injection à l'anode.
- Détection à la cathode, par spectroscopie UV à 260 nm (longueur d'onde d'absorption maximum de l'oligonucléotide).

Le conjugué copolymère-oligonucléotide est ensuite purifié par chromatographie par perméation de gel (CPG) dans les conditions suivantes :
- Colonne WATERS Ultra Hydrogel 500.
- Tampon phosphate 0,1 M pH = 6,8.
- Détection par spectroscopie UV à 260 nm.
- Débit 0,5 ml/min.

Après purification, le conjugué en solution saline est dialysé pendant une nuit à +4°C afin d'éliminer le sel. L'eau est éliminée à l'évaporateur à centrifugation sous vide. Le résidu sec est repris dans 1 ml d'eau distillée et stocké au congélateur à -20°C.

La liste des oligonucléotides utilisés pour le couplage sur un copolymère est donnée ci après.

Les caractéristiques des conjugués copolymère-oligonucléotide sont donnés dans le tableau 4 :

**TABLEAU 4**

| conjugué | copolymère | nom d'usage oligonucléotide | SEQ ID NO (a) | rendement (b) | Tr (c) | Me (d) | concentration (e) |
|---|---|---|---|---|---|---|---|
| CJOA | COPO3 | 1844 | 1 | 41 | 9,8 | 9,9 | 2,5 |
| CJOB | COPO3 | 1978 | 2 | 52 | 9,6 | 9,9 | 3,8 |
| CJOC | COPO3 | 1854 | 3 | 48 | 10,1 | 10,1 | 8,7 |

(a) Les oligonucléotides ont les séquences suivantes (de 5' vers 3'): 
SEQ ID NO1 :
TCATCCACCT GGCATTGGAC TGCCATAACC ATGAGTG . . . . . . . . . . . . . . . . . 37
SEQ ID NO2 :
TACTCACCAG TCACAGAAAA GC . . . . . . . . . . . . . . . . . . . . . . . . . . . . . 22
SEQ ID NO3 :
GATGAGCTAT ATGAGAACGG TA . . . . . . . . . . . . . . . . . . . . . . . . . . . . . 22
   Ces oligonucléotides possèdent à leur extrémité 5' un bras aliphatique avec une fonction NH₂, préparé selon la méthode décrite dans la demande de brevet WO 91/19812.
(b) Le rendement de couplage, calculé par l'intégration des pics en CPG, est le rapport, par CLHP, de la surface du pic du conjugué sur la somme des surfaces du pic du conjugué et de l'oligonucléotide non couplé. Il est donné en %.
(c) Tr représente le temps de rétention en CPG dans les conditions décrites dans le paragraphe précédent. Il est donné en minutes.
(d) Me représente le temps de rétention donné en minutes dans les conditions de séparation en électrophorèse capillaire décrites dans le paragraphe précédent.
(e) La concentration de l'oligonucléotide est donnée en picomoles par microlitres, par spectrométrie U.V., en mesurant l'absorbance à 260 nm, connaissant le coefficient d'extinction molaire de l'oligonucléotide à cette même longueur d'onde.

### EXEMPLE 2 : Détection d'un fragment d'acides nucléiques par protocole sandwich en utilisant des oligonucléotides couplés à des copolymères comme amplification de détection :

Une P.C.R. est effectuée selon le protocole standard tel que décrit dans "PCR PROTOCOLS: a guide to methods and applications", édité par M.A. Innis, D.H. Gelfand, J.J. Sninsky, T.J. White, Academic Press. La cible est un plasmide ADN pBR 322 (référence D4904, SIGMA) contenant 4363 paires de bases.

Le plasmide est en solution dans un tampon Tris 10 mM, pH 8,0. Les deux amorces utilisées pour l'amplification sont TEM1 (SEQ ID NO 4) et TEM2 (SEQ ID NO 5) :

SEQ ID NO 4 :
CCCCGAAGCG TTTTC. . . . . . . . . . . . . . . . . . . . . . . . . . . . 15
SEQ ID NO 5 :
CGGTATGGTT TGCTGCT. . . . . . . . . . . . . . . . . . . . . . . . . . . 17

Le fragment de PCR produit est contrôlé sur gel d'acrylamide à 5 %. Il contient 347 paires de bases.

Le fragment de PCR est dénaturé avant utilisation en ajoutant à 100 microlitres de solution le contenant, 10 microlitres de soude 2N. L'ensemble est neutralisé 5 minutes après par l'addition de 10 microlitres d'acide acétique 2N.

Dans deux plaques de microtitration en polystyrène NUNC référence 439454 sont déposés 100 microlitres d'une solution de l'oligonucléotide 1978 ((SEQ ID NO 2) à la concentration de 150 nM dans du tampon PBS3X (phosphate de sodium 0,15 M, chlorure de sodium 0,45 M, pH 7,5). L'incubation s'effectue durant deux heures à la température de 37°C.

Après 3 lavages des plaques par 300 microlitres de tampon PBS-Tween (phosphate de sodium 50 mM, chlorure de sodium 150 mM, contenant 0,5 ml/l de Tween 20 référence MERCK 822184), 100 microlitres de fragment PCR dénaturé, dilué en tampon PEG (phosphate de sodium 0,1 M, chlorure de sodium 0,5M, pH 7,0 contenant 0,14 mg/ml d'ADN de saumon, 20g/l de PEG 4000 référence MERCK 807490 et 6,5g de Tween 20 référence BIORAD 170-6531) sont ajoutés dans les puits des plaques de microtitration. Les plaques sont incubées une heure à 37°C puis lavées par 3 fois 300 microlitres de PBS-Tween.

Dans chaque puits d'une plaque sont ajoutés 100 microlitres de sonde de détection, oligonucléotide 1844 (SEQ ID NO 1) couplé à la phosphatase alcaline, à la concentration de 15 nM en oligonucléotides en tampon PEG. La sonde de détection 1844 contient une séquence complémentaire de la cible PCR. Elle est marquée à la phosphatase alcaline (akp) tel que décrit dans la demande de brevet WO 91/19812. La plaque est incubée une heure à 37°C puis lavée par 3 fois 300 microlitres de PBS-Tween. Cette plaque correspond à un protocole sandwich direct.

En parallèle, dans chaque puits de la deuxième plaque de microtitration sont déposés 100 microlitres d'un conjugué oligonucléotide-copolymère CJOA dans du tampon PEG. Ce conjugué est un intermédiaire dans le système sandwich qui permet une augmentation de sensibilité. La plaque est incubée une heure à 37°C puis lavée par 3 fois 300 microlitres de PBS-Tween. Dans chaque puits de la plaque sont ajoutés 100 microlitres de la sonde de détection oligonucléotide 1841 (SEQ ID NO 6) couplé à la phosphatase alcaline à la concentration de 15 nM en oligonucléotides en tampon PEG. Cette plaque correspond à un protocole avec amplification de signal par l'intermédiaire du copolymère-oligonucléotide.

SEQ ID NO 6 :
GTCCAATGCC AGGTGGATGA . . . . . . . . . . . . . . . . . . . . . . . . . 20

La sonde de détection est complémentaire d'une portion de l'oligonucléotide 1844 couplé sur le copolymère. Elle est marquée à la phosphatase alcaline tel que décrit dans la demande de brevet WO 91/19812.

La plaque est incubée une heure à 37°C puis lavée par 3 fois 300 microlitres de PBS-Tween.

Les 2 plaques sont traitées ensuite de façon identique.
Dans chaque puits sont ajoutés 100 microlitres de substrat PNPP (Para Nitro Phényl Phosphate, référence SIGMA N 2765) ; pour cela, une pastille de 20 mg de substrat est reprise par 10 ml de tampon diéthanolamine 1M, 0,5 mM MgCl₂ pH 9,8. Après 20 minutes de réaction, l'activité enzymatique est bloquée par 100 microlitres de NaOH 1N.

La lecture de la densité optique est effectuée à une longueur d'onde de 405 nm sur un lecteur de plaque AXIA MICROREADER BIOMERIEUX.

Les résultats (valeurs de densité optique) obtenus sont résumés dans le tableau 5 suivant:

**TABLEAU 5**

| dilution de la PCR | détection sandwich directe | oligonucléotide-copolymère en détection |
|---|---|---|
| 0 | 12 | 10 |
| dil 1/100 000 | 12 | 15 |
| dil 1/10 000 | 10 | 16 |
| dil 1/1000 | 11 | 30 |
| dil 1/100 | 20 | 137 |
| dil 1/10 | 160 | 1110 |

L'erreur de mesure est d'environ 10 %.

Les valeurs obtenues correspondent aux unités de densité optique mesurées sur un AXIA MICROREADER (AXIA marque déposée BioMérieux).

Le puits ne contenant pas de fragment PCR (0) sert de témoin négatif. Avec le système direct (sans amplification), le fragment de PCR est détecté pour une dilution au 1/10ème. Avec le système copolymère-oligonucléotide, le fragment de PCR est détecté pour une dilution inférieure au 1/100ème, le signal obtenu est multiplié par un facteur 10. La sensibilité du test est augmentée d'un facteur supérieur à 10. Le bruit de fond n'est pas augmenté par l'amplification de signal.

### EXEMPLE 3: Détection d'un fragment d'acides nucléiques par protocole sandwich en utilisant des oligonucléotides couplés à des copolymères en détection et en capture :

Le protocole est identique à celui de l'exemple 2 à l'exception du fait que dans chaque puits d'une des plaques de microtitration, est déposé, à la place de l'oligonucléotide 1978, le conjugué CJOB (oligonucléotide 1978 (SEQ ID NO 2) couplé au copolymère COPO3) à une concentration en oligonucléotide de 150 nM dans du PBS 3X, comme sonde de capture.

Le copolymère CJOA est utilisé en tant que système d'amplification de détection comme dans l'exemple 2.

L'autre plaque, qui servira de contrôle pour mesurer le gain en sensibilité, est préparée de la même manière que dans l'exemple 2 (détection directe).

Les résultats ( valeurs de densité optique ) obtenus sont résumés dans le tableau 6 :

**TABLEAU 6**

| dilution de la PCR | détection sandwich directe | détection avec 2 copolymères en capture et en détection |
|---|---|---|
| 0 | 10 | 70 |
| dil 1/100 000 | 10 | 90 |
| dil 1/10 000 | 11 | 101 |
| dil 1/1000 | 21 | 298 |
| dil 1/100 | 28 | 705 |
| dil 1/10 | 198 | >2500 |

L'erreur de mesure est d'environ 10 %.

Les valeurs obtenues correspondent aux unités de densité optique mesurées sur un AXIA MICROREADER (AXIA marque déposée BioMérieux).

La valeur 2500 correspond à la saturation du lecteur de microplaques.

Le puits ne contenant pas de fragment PCR (0) sert de témoin négatif. Avec le système oligonucléotide-phosphatase (sans amplification), le fragment de PCR est détecté pour une dilution au 1/10ème. Avec le système copolymère-oligonucléotide en capture et en détection, le fragment de PCR est détecté pour une dilution au 1/1000ème. La sensibilité du test est augmentée d'un facteur 100.

Si l'on compare ce gain avec celui obtenu dans l'exemple 2 qui était d'environ 10 fois, il apparait que la combinaison de deux conjugués oligonucléotide-bipolymère à la fois en détection et en capture est encore plus performante non seulement en ce qui concerne le gain en sensibilité mais également en ce qui concerne le rapport signal sur bruit de fond.

### EXEMPLE 4 :

Détection d'alpha foeto protéine (AFP) par protocole sandwich en utilisant un copolymère-oligonucléotide comme intermédiaire en détection pour augmenter le signal.

Le gain en sensibilité est apprécié en comparant à un protocole sandwich immunologique classique.

Dans deux plaques de microtitration en polystyrène NUNC référence 439454, sont déposés 100 microlitres d'une solution de l'anticorps monoclonal anti alpha foeto protéine (référence P3F11G9 BIOMERIEUX) dilué à la concentration de 10 microgrammes/ml en tampon carbonate de sodium 50mM (Na₂ CO₃ PROLABO 27771290-NaHCO₃ PROLABO 27778293) pH 9,6. L'incubation s'effectue durant deux heures à la température de 37°C. Les deux plaques sont lavées par 300 microlitres de tampon PBS 1X (phosphate de sodium 50 mM, chlorure de sodium 150 mM contenant 0,5 ml/l de Tween 20 référence MERCK 822184).

Dans une plaque (sandwich immunologique classique), 50 microlitres d'antigène alpha foeto protéine (référence BEHRING OTOD 02/03) dilué en tampon PBS Tween contenant 10 % de sérum de cheval et 50 microlitres d'un deuxième anticorps polyclonal de chèvre anti alpha foeto protéine (référence CH109-1981 BIOMERIEUX) couplé à la peroxydase, (Boehringer Mannheim, référence 814393) selon les méthodes classiques, à la concentration de 2 mg/l en tampon PBS-Tween contenant 10 % de sérum de cheval sont ajoutés par puits. La plaque est incubée une heure à 37°C puis lavée par 3 fois 300 microlitres de PBS-Tween.

En parallèle dans la deuxième plaque (détection avec amplification de signal), sont déposés 50 microlitres d'antigène alpha foeto protéine (référence BEHRING OTOD 02/03) dilué en tampon PBS Tween contenant 10 % de sérum de cheval puis 50 microlitres du deuxième anticorps polyclonal anti alpha foeto protéine (référence CH109-1981, BioMérieux) couplé à un oligonucléotide 1614 (SEQ ID NO 7) dilué en tampon PEG (phosphate de sodium 0,1M, chlorure de sodium 0,5M, pH 7,0 contenant 0,14 mg/ml d'ADN de saumon , 20g/l de PEG 4000 (référence MERCK 807490) et 6,5g de Tween 20 (référence BIORAD 170-6531)).

La concentration de la solution de PEG contenant l'anticorps couplé à l'oligonucléotide 1614 est de 2 mg/l en anticorps.

SEQ ID NO 7 :
TACCGTTCTC ATATAGC. . . . . . . . . . . . . . . . . . . . . . . . 17

Le couplage entre l'anticorps et l'oligonucléotide 1614 est effectué selon la même méthode que décrite pour le couplage oligonucléotide peroxydase dans la demande de brevet WO 91/19812.

La plaque est incubée une heure à 37°C puis lavée par 3 fois 300 microlitres de PBS-Tween. 100 microlitres d'une solution d'un conjugué oligonucléotide-copolymère CJOC à la concentration de 150nM en oligonucléotide dans du tampon PEG sont ajoutés dans les puits de la plaque de microtitration. La plaque est incubée une heure à 37°C, puis lavée par 3 fois 300 microlitres de PBS-Tween.

Dans chaque puits de la plaque, sont ajoutés 100 microlitres de sonde de détection oligonucléotide 1614 (SEQ ID NO 7) couplé à la peroxydase (demande de brevet WO 91/19812) à la concentration de 15 nM en oligonucléotides en tampon PEG. La plaque est incubée une heure à 37°C puis lavée par 3 fois 300 microlitres de PBS-Tween.

La révélation de l'activité enzymatique est la même pour les deux plaques.

Dans chaque puits sont ajoutés 100 microlitres de solution de substrat OPD (Ortho Phénylène Diamine, référence SIGMA P7288). Pour préparer cette solution de substrat, une pastille de 20 mg de substrat est reprise par 10 ml de tampon citrate pH 4,93(0,055 M acide citrique, 0,1M Na₂ HPO₄). Après 15 minutes de réaction à 37°C, l'activité enzymatique est bloquée par 100 microlitres d'acide sulfurique 1N.

La lecture de la densité optique est effectuée à une longueur d'onde de 492 nm sur un lecteur de plaque AXIA MICROREADER BIOMERIEUX.

Les résultats (valeurs de densité optique) obtenus sont résumés dans le tableau 7 :

**TABLEAU 7**

| concentration en antigène AFP (ng/ml) | détection immunologique classique | copolymère-oligonucléotide en détection |
|---|---|---|
| 0 | 55 | 110 |
| 0,05 | 60 | 110 |
| 0,10 | 60 | 250 |
| 0,50 | 60 | 420 |
| 1,0 | 120 | 670 |
| 2,5 | 250 | 1620 |
| 5,0 | 370 | >2500 |
| 10,0 | 630 | >2500 |

L'erreur de mesure est d'environ 10 %.

Les valeurs obtenues correspondent aux unités de densité optique mesurées sur un AXIA MICROREADER (marque déposée BioMérieux).

La valeur 2500 correspond à la saturation du lecteur de microplaques.

Les résultats obtenus montrent une augmentation très forte de la sensibilité du test avec l'emploi du copolymère-oligonucléotide. Une détection de 0,1 ng/ml d'alpha foeto protéine est possible avec le système d'amplification utilisant le copolymère-oligonucléotide, l'augmentation du signal obtenu étant d'un facteur 2 par rapport au puits ne contenant pas d'antigène (puits 0). Sans amplification (détection immunologique classique), la détection de l'AFP commence à 1 ng/ml.

Dans cet exemple, l'anticorps de détection est un anticorps polyclonal.

### EXEMPLE 5 :

Détection d'alpha foeto protéine par protocole sandwich en utilisant des oligonucléotides couplés à des copolymères en détection et/ou en capture.

Le gain en sensibilité est apprécié en comparant à un protocole sandwich immunologique classique.

Le protocole sandwich immunologique classique est mené de la même manière que celui décrit dans l'exemple 4 à l'exception du fait qu'on utilise un anticorps monoclonal (P7H10B7, BioMérieux) couplé à la peroxydase à la place de l'anticorps polyclonal (CH109-1981, BioMérieux) en détection.

Le protocole utilisant des oligonucléotides couplés à des copolymères en détection est identique à celui de l'exemple 4 à l'exception du fait qu'on utilise, comme anticorps de détection, un anticorps monoclonal (P7H10B7, BioMérieux) à la place de l'anticorps polyclonal (CH109-1981, BioMérieux).

Le protocole utilisant des oligonucléotides couplés à des copolymères en détection et en capture est le suivant :

Dans une plaque de microtitration, sont déposés 100 microlitres d'un conjugué oligonucléotide-copolymère CJOA à la concentration de 150 nM en oligonucléotide dans du tampon PBS3X. La plaque est incubée deux heures à 37°C puis rincée par 3 fois 300 microlitres de PBS-Tween.

Puis sont déposés 100 microlitres d'anticorps monoclonal anti alpha foeto protéine (référence P3F11G9, BIOMERIEUX) couplé, comme décrit dans l'exemple 4, à l'oligonucléotide 1841 (SEQ ID NO 6). Cet anticorps couplé à l'oligonucléotide 1841 est dilué en tampon PEG à la concentration de 2 mg/l en anticorps. La plaque est incubée une heure à 37°C puis lavée par 3 fois 300 microlitres de PBS-Tween.

L'antigène AFP puis l'anticorps monoclonal (P7H10B7, BioMérieux) couplé à un oligonucléotide 1614 (SEQ ID NO 7) sont ajoutés comme décrit dans l'exemple 4.

Les étapes de réaction avec le conjugué CJOC, avec l'oligonucléotide 1614 couplé à la peroxydase et de révélation par le substrat OPD sont identiques à celles de l'exemple 4.

Les résultats ( valeurs de densité optique ) obtenus sont résumés dans le tableau 8 :

**TABLEAU 8**

| concentration en antigène AFP (ng/ml) | détection immunologique classique | copolymère-oligonucléotide en détection | copolymère-oligonucléotide en capture et détection |
|---|---|---|---|
| 0 | 33 | 155 | 110 |
| 1 | 35 | 175 | 250 |
| 5 | 70 | 651 | 650 |
| 10 | 140 | 1230 | 1425 |
| 50 | 545 | >2500 | >2500 |
| 100 | 1025 | >2500 | >2500 |

L'erreur de mesure est d'environ 10 %.

Les valeurs obtenues correspondent aux unités de densité optique mesurées sur un AXIA MICROREADER (AXIA : marque déposée, BioMérieux).

La valeur 2500 correspond à la saturation du lecteur de microplaques.

On observe un gain en sensibilité important en comparant la détection immunologique classique et l'utilisation d'un copolymère-oligonucléotide en détection. Les résultats vont dans le même sens que pour l'exemple 4 avec cette fois un anticorps monoclonal à la place d'un anticorps polyclonal en détection. Le rapport signal-bruit pour une quantité d'AFP de 5 ng/ml est d'environ 2 pour la détection immunologique classique alors qu'il est de 4,2 pour le système copolymère-oligonucléotide en détection.

Comme montré précédemment pour les acides nucléiques dans l'exemple 3, on observe une amélioration du grain en sensibilité en utilisant un copolymère-oligonucléotide en détection et en capture.

En effet, pour une concentration de 1 ng/ml d'AFP, le rapport signal-bruit est de 2,3, ce qui permet de détecter cette concentration.

Ce résultat est obtenu, entre autres, grâce à une réduction du bruit de fond, ce qui montre l'intérêt de la méthode puisque le bruit de fond est un problème essentiel dans tous les systèmes d'amplification du signal.

De manière générale, les bruits de fond observés avec un oligonucléotide-bipolymère en détection et/ou en capture sont faibles (voir les exemples 2 à 4)

### EXEMPLE 6 : influence du nombre d'oligonucléotides couplés sur le conjugué copolymère-oligonucléotide pour l'amplification de la détection :

Dans le procédé décrit, l'amplification du signal est relié directement au nombre d'oligonucléotides fixés sur le copolymère.

En effet, dans le procédé sandwich classique, pour une molécule cible hybridée sur une sonde de capture, une sonde de détection portant un seul traceur, par exemple enzymatique, est susceptible de s'hybrider.

En utilisant un copolymère-oligonucléotide comme sonde intermédiaire, le nombre de sondes de détection et donc le nombre de traceurs susceptible de s'hybrider avec une molécule cible est en théorie égal au nombre de sondes couplées sur le copolymère moins une (celle qui s'hybride sur la cible).

### Greffage d'un nombre variable d'oligonucléotides sur un copolymère :

Une solution aqueuse contenant 15 nmoles de l'oligonucléotide 12 (SEQ ID NO 8) est évaporée à sec dans un tube Eppendorf de 1,5 ml.

SEQ ID NO 8 :
TCATCCACCT GGCATTGGAC . . . . . . . . . . . . . . . . . . . . . . . . . . . . . . 20

Le résidu sec est repris dans x₁ microlitres de tampon borate 0,1 M, pH 9,3.Sont ajoutés dans la solution, y₁ microlitres de DMF puis z₁ microlitres du copolymère COPO3 (tableau 1) à 1 mg/ml dans le DMF. Après 3 heures de réaction à 37°C, le solvant est éliminé à l'évaporateur sous vide. Le résidu sec est repris par 50 microlitres d'eau et analysé en perméation de gel dans les conditions décrites dans l'exemple 1.

Le tableau 9 résume les conditions de couplage pour les différents conjugués préparés et le tableau 10 les caractéristiques des conjugués préparés.

**TABLEAU 9**

| conjugués | x₁ | y₁ | z₁ | stoechiométrie |
|---|---|---|---|---|
| CJOD1 | 30 | 255 | 15,0 | 1/4 |
| CJOD2 | 30 | 248 | 22,5 | 1/6 |
| CJOD3 | 30 | 225 | 45 | 1/12 |
| CJOD4 | 30 | 180 | 90 | 1/24 |
| CJOD5 | 30 | 45 | 225 | 1/60 |
| CJOD6 | 42 | 0 | 375 | 1/100 |

x₁, y₁ et z₁ sont donnés en microlitres.
La stoechiométrie S représente le rapport entre le nombre de moles d'oligonucléotides introduits et le nombre de moles de fonctions NHS disponibles pour le couplage.

**TABLEAU 10**

| Conjugués | Rendement de couplage R | Temps de rétention | N | Concentration |
|---|---|---|---|---|
| CJOD1 | 49 | 9,80 | 31,5 | 3,4 |
| CJOD2 | 53 | 9,66 | 22,7 | 4,5 |
| CJOD3 | 57 | 9,72 | 12,2 | 5,8 |
| CJOD4 | 59 | 9,51 | 6,3 | 5,4 |
| CJOD5 | 55 | 9,70 | 2,4 | 5,3 |
| CJOD6 | 60 | 9,50 | 1,6 | 4,6 |

Le temps de rétention HPLC (en minutes), le rendement de couplage R (en %), la concentration (en nmoles/ml) des conjugués sont calculés de la même manière que dans le tableau 4 de l'exemple 1.

N représente le nombre de moles d'oligonucléotides par mole de copolymère. Le nombre théorique Nt d'oligonucléotides par chaîne est calculé en connaissant la stoechiométrie S de la réaction par rapport au nombre de fonctions NHS disponibles sur le copolymère (257 fonctions pour le COPO3 : voir tableau 2) ; Nt = 257 x S.

N est calculé selon la formule : N = Nt x R.

### Comparaison de l'efficacité des différents conjugués en amplification de détection :

Le gain en sensibilité est mesuré en comparant un protocole utilisant les différents conjugués oligonucléotide-copolymère comme intermédiaires en détection. Un essai de référence sans copolymère-oligonucléotide comme intermédiaire est réalisé comme décrit dans l'exemple 2 (détection sandwich directe).

Le protocole avec amplification de signal par l'intermédiaire copolymère-oligonucléotide est identique à celui décrit dans l'exemple 2 à l'exception du fait que l'oligonucléotide de capture déposé au fond de la plaque est l'oligonucléotide 474 (SEQ ID NO 9).

SEQ ID NO 9 :
AAAAAAAAAA AAAAAAAAAA AAAAAAAAAA. . . . . . . . . . . . . . . . . . . . . . . . . 30

La cible n'est pas un fragment PCR mais un oligonucléotide synthétique 469 (SEQ ID NO 10) simple brin et qui donc n'a pas besoin d'être dénaturé par la soude.

SEQ ID NO 10:
TTTTTTTTTT TTTTTTTTTT GTCCAATGCC AGGTGGATGA . . . . . . . . . . . . 40

Cette cible est dilué dans le tampon PEG de 5000 pg/ml à 1 pg/ml.

Une partie de cette cible est complémentaire de l'oligonucléotide 12 fixé sur le copolymère et une autre partie de l'oligonucléotide 474.

Les différents copolymères CJOD1 à CJOD6 sont déposés en parallèle dans des puits de la plaque de microtitration.
La sonde de détection est l'oligonucléotide 1841 (SEQ ID NO 6) couplé à la peroxydase de raifort.

Le substrat de l'enzyme est l'OPD tel que décrit dans l'exemple 4, avec une lecture à 492 nm sur un lecteur AXIA MICRORADER. (AXIA marque déposée BIOMERIEUX).

### Protocole sandwich direct:

Capture par oligonucléotide 474 (SEQ ID NO 9)

Cible synthétique 469 (SEQ ID NO 10)

Détection par sonde de détection 12 (SEQ ID NO 8) marquée à la peroxydase.

Le tableau 11 résume les résultats obtenus avec les différents copolymères-oligonucléotide.

**TABLEAU 11**

| concentration de cible (pg/ml) | CJOD1 | CJOD2 | CJOD3 | CJOD4 | CJOD5 | CJOD6 | direct |
|---|---|---|---|---|---|---|---|
| 5000 | 2500 | 2500 | 2500 | 2500 | 2500 | 2500 | 2500 |
| 1000 | 2500 | 2500 | 2500 | 2500 | 2500 | 1536 | 601 |
| 50 | 2500 | 2500 | 2500 | 2500 | 1461 | 995 | 370 |
| 50 | 1661 | 1253 | 1213 | 590 | 549 | 480 | 5 |
| 5 | 627 | 543 | 455 | 243 | 185 | 184 | 0 |
| 1 | 519 | 458 | 332 | 206 | 0 | 0 | 0 |

L'erreur de mesure est d'environ 10 %.

Les valeurs obtenues correspondent aux unités de densité optique mesurées sur un AXIA MICROREADER (AXIA marque déposée BioMérieux).

Le bruit de fond déterminé par une essai sans cible pour chaque conjugué est soustrait des valeurs obtenues pour les différents conjugués aux différentes concentrations de cible.

On constate que le nombre de moles d'oligonucléotide par mole de copolymère est en relation avec le gain obtenu en amplification de signal. Les valeurs obtenues pour la plus faible concentration de cible de 1 pg/ml sont croissantes quand le nombre d'oligonucléotides fixés sur le copolymère augmente. Cette concentration n'est pas détectée avec CJOD5 et CJOD6 où seulement 1,6 et 2,4 oligonucléotides, respectivement, sont présents par mole de copolymère.

Même avec ces derniers conjugués il y a un gain en sensibilité par rapport à la détection sandwich directe.

### EXEMPLE 7 : Synthèse et caractérisation des conjugués avec des copolymères à base d'AMVE:

### Définition du copolymère AMVE.

Le copolymère AMVE employé est un copolymère d'anhydride maléique et d'éther méthylvinylique, commercialisé par la société Polyscience sous la référence 03102. Sa masse moléculaire est de 67000 et c'est un copolymère alterné constitué de 50 % de chacun des deux motifs monomères.

### Protocole de couplage des oligonucléotides sur copolymère AMVE.

25 nmoles d'oligonucléotide sont séchés à l'évaporateur à centrifugation sous vide puis repris par 7 microlitres de tampon borate de sodium 0,1M, NaCl 0,5 M, pH 9,3. On ajoute ensuite à cette solution 143 microlitres de diméthylsulfoxyde anhydre puis 10 microlitres de solution de copolymère dans le diméthylsulfoxyde anhydre à la concentration de 15 g/l pour les conjugués CJOE, CJOG, CJOI ou 5 g/l pour les conjugués CJOF, CJOH, CJOI. Après 6h d'agitation à 37°C, on ajoute au milieu réactionnel 100 microlitres de tampon hydrogénocarbonate de sodium 100 mM pH 8,2 qu'on laisse réagir 30 minutes afin d'hydrolyser les sites du copolymère éventuellement encore disponibles. Les solvants sont éliminés à l'évaporateur à centrifugation sous vide puis les conjugués sont repris par 200 microlitres d'eau distillée et purifiés par chromatographie haute performance sur une colonne Waters Ultra Hydrogel 500 en tampon phosphate 0,1 M, pH 6,8.

Les résultats obtenus sont résumés dans le tableau 12 ci-dessous :

**TABLEAU 12**

| conjugué | nom d'usage de l'oligonucléotide | SEQ ID NO (a) | rendement (b) | Tr (c) | concentration (e) | N |
|---|---|---|---|---|---|---|
| CJOE | 1844 | 1 | 34 | 10,57 | 1,32 | 2 |
| CJOF | 1844 | 1 | 38 | 9,88 | 1,76 | 7 |
| CJOG | 1854 | 3 | 59 | 10,17 | 2,74 | 6 |
| CJOH | 1854 | 3 | 47 | 9,90 | 2,18 | 14 |
| CJOI | 1843 | 11 | 52 | 10,71 | 1,98 | 4 |
| CJOJ | 1843 | 11 | 30 | 9,87 | 1,39 | 7 |

N représente le nombre d'oligonucléotides par chaîne de copolymère.
(a) L'oligonucléotide 1843 (SEQ ID NO 11) a pour séquence : 
SEQ ID NO 11 :

CGCTTTTTTG CACAACATGG GGGATCATG. . . . . . . . . . . . . . . . . . 29
   Ces oligonucléotides possèdent à leur extrémité 5' un bras aliphatique avec une fonction amine préparé selon la méthode décrite dans WO 91/19812.
(b) Le rendement de couplage, calculé par l'intégration des pics en CPG, est le rapport, par CLHP, de la surface du pic du conjugué sur la somme des surfaces du pic du conjugué et de l'oligonucléotide non couplé. Il est donné en %.
(c) Tr représente le temps de rétention en CPG dans les conditions décrites dans l'exemple 1. Il est donné en minutes.
(e) La concentration de 1' oligonucléotide est donnée en picomoles par microlitres, par spectrométrie U.V., en mesurant l'absorbance à 260 nm, connaissant le coefficient d'extinction molaire de l'oligonucléotide à cette même longueur d'onde.

### EXEMPLE 8:

Des plaques de microtitration NUNC (Maxisorb) sont revêtues par un anticorps monoclonal (référence bioMérieux, P3F11G9) anti alphafoeto protéine dilué à 10 microgrammes/millilitre dans du tampon carbonate 0,05 M pH 9,7. L'incubation dure indifféremment soit 2 heures à 37°C, soit 18 heures à température ambiante, soit 56 heures à la température de +4°C. L'excès de réactif est éliminé et les plaques sont lavées avec un tampon PBS-Tween. 100 microlitres d'une solution de caséine à un gramme/litre sont ensuite déposés dans chaque puits et les plaques sont incubées une heure à 37°C, puis lavées comme précédemment. 50 microlitres de solution d'antigène alpha foeto protéine Behring à diverses dilutions dans un tampon de PBS-Tween contenant 10 % de sérum de cheval sont introduits dans les puits, puis on ajoute 50 microlitres d'une solution de conjugué oligonucléotide 1614 (SEQ ID NO 7), complémentaire de la séquence du conjugué CJOG ou CJOH, couplé à 1' anticorps polyclonal anti alphafoeto protéine (référence CH109-1981, BioMérieux). Ce conjugué est dilué dans un tampon PBS-Tween contenant 10 % de sérum de cheval. Après une heure d'incubation à 37°C et lavages au PBS Tween, on ajoute dans chaque puits 100 microlitres de solution de conjugué copolymère-oligonucléotides dilué dans un tampon PEG. Après une heure d'incubation à 37°C, puis lavages, on ajoute dans chaque puits 100 microlitres de solution en PEG de la sonde de détection : oligonucléotide 1841(SEQ ID NO6) - peroxydase que l'on incube une heure à 37°C. Après lavage de l'excès de réactifs, on introduit dans chaque puits 100 microlitres de solution de substrat OPD. Après 7 minutes de révélation à température ambiante, on bloque la réaction par 0,1 ml de solution normale d'acide sulfurique (voir exemple 4 pour les détails).

Les plaques de référence non amplifiées sont réalisées de façon identique en omettant l'étape du polymère. Les résultats obtenus sont reportés dans le tableau 13 ci-dessous :

**TABLEAU 13**

| Antigène AFP | détection immunologique classique | conjugué | conjugué |
|---|---|---|---|
| (ng/ml) | | CJOG | CJOH |
| 0 | 50 | 44 | 48 |
| 0,5 | 60 | 71 | 101 |
| 1 | 70 | 151 | 215 |
| 2,5 | 90 | 530 | 828 |
| 5 | 130 | 1600 | 2500 |
| 10 | 220 | 2500 | 2500 |
| 25 | 550 | 2500 | 2500 |
| 50 | 880 | 2500 | 2500 |

L'erreur de mesure est d'environ 10 %.

Les valeurs obtenues correspondent aux unités de densité optique mesurées sur un AXIA MICROREADER (marque déposée BioMérieux).

La valeur 2500 correspond à la saturation du lecteur de microplaques.

Cet exemple montre une augmentation très forte de la sensibilité avec l'emploi d'un conjugué oligonucléotide-AMVE comme intermédiaire de détection. La concentration d'AFP pour laquelle le rapport signal/bruit de fond est supérieur à 2 est de 5 ng/ml pour la détection immunologique classique, de 0,5 ng/ml pour le conjugué CJOG et de 0,1 ng/ml pour CJOH. Comme dans l'exemple 6 pour le copolymère NVPNAS, le gain en sensibilité est amélioré en augmentant le ratio N nombre de moles d'oligonucléotide par mole de copolymère. N est égal à 6 pour CJOG et est égal à 14 pour CJOH.

### EXEMPLE 9:

Détection d'un fragment d'acides nucléiques par protocole sandwich sur automate VIDAS en utilisant des oligonucléotides couplés à des copolymères en détection et en capture. Comparaison d'un protocole 1 étape et d'un protocole 2 étapes.

Une P.C.R. est effectuée puis dénaturée tel que décrit dans l'exemple 2.

Le protocole suivant est effectué automatiquement sur l'automate VIDAS (marque déposée-commercialisé par la société BioMérieux SA-VITEK). Les étapes suivantes sont réalisées automatiquement.

La réaction est conduite dans un support conique appelé SPR ("Solid Phase Receptacle"), support conique réalisé à partir d'un matériau vendu sous la dénomination K résine, qui est un copolymère butadiène-styrène, et commercialisé par la société BioMérieux-Vitek (USA). Les divers réactifs sont disposés dans la barrette et les différentes étapes se déroulent dans le SPR qui fait office de pipette. La réaction d'hybridation sandwich décrite dans le protocole ci-dessous se produit sur la paroi interne du cône.

Sur la surface interne du SPR, est fixé passivement le conjugué oligonucléotide 1978-copolymère (CJOB, tableau 4). La concentration utilisée est de 0,15 nmole/ml d'oligonucléotides dans un volume de 300 microlitres d'une solution de PBS 4x (phosphate de sodium 200 mM pH 7,0, NaCl 600 mM). Après une nuit à température ambiante ou deux heures à 37°C, les cônes sont lavés 2 fois par une solution de PBS Tween , puis séchés sous vide.

La barrette contient l'ensemble des réactifs nécessaires à la détection, dans des puits indépendants, c'est à dire:
puits 2: 100 microlitres d'une solution à 0,015 nmoles/ml de conjugué CJOA (1844 couplé au copolymère NVPNAS) dans un tampon PEG (phosphate de sodium 150 mM, NaCl 450 mM, pH 7,0 + ADN de sperme de saumon 0,14 mg/ml (Sigma D 9156) + 20 g/l de PEG 4000 (Merck 807490) + 6,5 g/l de tween 20 (Biorad 170-6531)),
puits 3 : 200 microlitres d'une solution à 0,045 nmoles/ml dans le tampon PEG de la sonde de détection oligonucléotide 1841-phosphatase alcaline. La sonde de détection est préparée comme décrit dans WO 91/19812.
puits 4 et 5 : 2 fois 600 microlitres de solution de lavage PBS Tween,
puits 6: 300 microlitres de substrat MUP (méthyl-4 umbelliféryl phosphate) en solution dans un tampon diéthanolamine

Dans le premier puits (puits 1) de la barrette, est déposé 200 microlitres de la solution de cible (fragment PCR dilué) dans un tampon PEG. Après incubation 45 minutes du cône par le mélange, reconstitué dans le puits 2, de la cible (110 microlitres du puits 1), de la sonde de détection et du conjugué copolymère-oligonucléotide pour l'amplification (110 microlitres du puits 3), le cône est lavé 2 fois par une solution de PBS Tween. 250 microlitres de substrat sont aspirés dans le cône pendant 15 minutes, puis relâchés dans une cuvette de lecture. L'appareil mesure le signal fluorescent exprimé en RFU (unités relatives de fluorescence) de la cuvette.

En parallèle, un protocole identique mais avec 2 incubations séparées est réalisé. La première incubation de 45 minutes comprend le fragment PCR neutralisé et l'oligonucléotide 1844 couplé à un polymère NVPNAS. Après lavage, la sonde de détection 1841-phosphatase alcaline est incubée 45 minutes.

Les résultats sont représentés dans le tableau 14.

**TABLEAU 14**

| dilution du fragment PCR | 2 ÉTAPES D'INCUBATION | 1 ETAPE D'INCUBATION |
|---|---|---|
| 1/10 | 10060 | 8915 |
| 1/100 | 2960 | 1575 |
| 1/1 000 | 780 | 595 |
| 1/10 000 | 470 | 495 |
| 1/100 000 | 355 | 360 |
| 0 | 348 | 338 |

Les niveaux de détection sont exprimés en RFU (unités relatives de fluorescence).

L'erreur de mesure est d'environ 10 %.

Le bruit de fond de l'essai est mesuré en ne mettant pas de cible (essai 0).

Cet exemple montre que la limite de détection est la même dans les deux cas. Il est donc possible d'effectuer l'amplification de signal par le copolymère de détection sans rallonger les temps de manipulation et sans augmenter le bruit de fond en utilisant un protocole en 1 étape. Cet exemple montre aussi qu'il est possible d'adapter l'amplification de signal sur des systèmes différents de la microplaque et notamment sur un autre type de phase solide puisque la K résine qui compose le cône SPR est différente de celle de la microplaque qui est en polystyrène.

### LISTE DES SEQUENCES

SEQ ID NO1:
TCATCCACCT GGCATTGGAC TGCCATAACC ATGAGTG

longueur : 37
type : acide nucléique
nombre de brins : simple
configuration : linéaire
source : synthèse chimique
caractéristique additionnelle : bras alkylamine à l'extrémité 5'
nom d'usage : 1844

SEQ ID NO2 :
TACTCACCAG TCACAGAAAA GC

longueur : 22
type : acide nucléique
nombre de brins : simple
configuration : linéaire
source : synthèse chimique
caractéristique additionnelle : bras alkylamine à l'extrémité 5'
nom d'usage : 1978

SEQ ID NO3 :
GATGAGCTAT ATGAGAACGG TA

longueur: 22
type : acide nucléique
nombre de brins : simple
configuration : linéaire
source : synthèse chimique
caractéristique additionnelle : bras alkylamine à l'extrémité 5'
nom d'usage : 1854

SEQ ID NO 4 :
CCCCGAAGCG TTTTC

longueur : 15
type : acide nucléique
nombre de brins : simple
configuration : linéaire
source: synthèse chimique
nom d'usage : TEM1

SEQ ID NO 5:
CGGTATGGTT TGCTGCT

longueur : 17
type : acide nucléique
nombre de brins : simple
configuration : linéaire
source : synthèse chimique
nom d'usage : TEM2

SEQ ID NO6 :
GTCCAATGCC AGGTGGATGA

longueur : 20
type : acide nucléique
nombre de brins : simple
configuration : linéaire
source : synthèse chimique
caractéristique additionnelle : bras alkylamine à l'extrémité 5'
nom d'usage : 1841

SEQ ID NO 7:
TACCGTTCTC ATATAGC

longueur : 17
type : acide nucléique
nombre de brins : simple
configuration : linéaire
source : synthèse chimique
caractéristique additionnelle : bras alkylamine à l'extrémité 5'
nom d'usage : 1614

SEQ ID NO 8 :
TCATCCACCT GGCATTGGAC

longueur : 20
type : acide nucléique
nombre de brins : simple
configuration : linéaire
source : synthèse chimique
caractéristique additionnelle : bras alkylamine à l'extrémité 5'
nom d'usage : 12

SEQ ID NO 9 :
AAAAAAAAAA AAAAAAAAAAA AAAAAAAAAAA

longueur: 30
type : acide nucléique
nombre de brins : simple
configuration : linéaire
source : synthèse chimique
nom d'usage : 474

SEQ ID NO 10 :
TTTTTTTTTT TTTTTTTTTT GTCCAATGCC AGGTGGATGA.

longueur : 40
type : acide nucléique
nombre de brins : simple
configuration : linéaire
source : synthèse chimique
nom d'usage : 469

SEQ ID NO 11 :
CGCTTTTTTG CACAACATGG GGGATCATG

longueur : 29
type : acide nucléique
nombre de brins : simple
configuration : linéaire
source : synthèse chimique
caractéristique additionnelle : bras alkylamine à l'extrémité 5'
nom d'usage : 1843

## Revendications

1. Nécessaire pour la détection d'une séquence nucléotidique d'intérêt, avec amplification de signal, comprenant au moins une sonde nucléotidique marquée avec un traceur, caractérisé par le fait qu'il contient, dans des conteneurs appropriés, un réactif soluble en milieu aqueux comprenant essentiellement un copolymère à squelette linéaire essentiellement carboné portant des substituants latéraux, dont la chaîne est constituée par un premier type de motifs répétitifs et par au moins un autre type de motifs répétitifs, dans lequel au moins une partie des motifs du premier type portent un substituant latéral comprenant une unité nucléotidique, un tel substituant latéral étant absent sur les autres types de motifs, chacune desdites unités nucléotidiques, toutes identiques, comprenant, au moins, une séquence nucléotidique capable d'hybridation avec ladite séquence d'intérêt et une séquence nucléotidique capable d'hybridation avec ladite sonde,
ledit réactif contenant en moyenne plus de deux desdites unités nucléotidiques, en équivalents molaires, par mole de polymère.

2. Nécessaire selon la revendication 1, caractérisé par le fait que, dans une même unité nucléotidique, lesdites séquences capables d'hybridation sont distinctes et non chevauchantes, et que ladite séquence nucléotidique capable d'hybridation avec la sonde, est alors une séquence choisie arbitrairement.

3. Nécessaire selon la revendication 1, caractérisé par le fait que, dans une même unité nucléotidique, l'une des dites séquences capables d'hybridation est incluse dans l'autre.

4. Nécessaire selon la revendication 3, caractérisé par le fait que, dans une même unité nucléotidique, lesdites séquences capables d'hybridation sont confondues, et la sonde contient alors une séquence qui est capable de s'hybrider avec la séquence complémentaire de ladite séquence d'intérêt.

5. Nécessaire selon l'une quelconque des revendications précédentes, caractérisé par le fait que ledit réactif contient en moyenne de 3 à 100, et en particulier de 5 à 50 desdites unités nucléotidiques, en équivalents molaires par mole de copolymère.

6. Nécessaire selon l'une quelconque des revendications précédentes, caractérisé par le fait que ledit copolymère est un bipolymère.

7. Nécessaire selon l'une quelconque des revendications précédentes, caractérisé par le fait que lesdits motifs répétitifs proviennent de la copolymérisation d'un premier monomère insaturé avec au moins un autre monomère insaturé.

8. Nécessaire selon la revendication précédente, caractérisé par le fait que ledit premier monomère insaturé porte une fonction réactive capable de réagir, soit directement, soit par l'intermédiaire d'un agent de couplage bifonctionnel, avec un précurseur de ladite unité nucléotidique pour former ledit substituant latéral.

9. Nécessaire selon la revendication précédente, caractérisé par le fait que ladite fonction réactive est choisie parmi les fonctions amine, imide, aldéhyde, époxy, thiol, halogénoalkyle ou acide carboxylique, l'acide carboxylique étant éventuellement activé sous forme d'halogénure d'acide, sous forme d'anhrydride ou sous forme d'ester.

10. Nécessaire selon la revendication précédente, caractérisé par le fait que ledit premier monomère est un dérivé d'acide carboxylique insaturé.

11. Nécessaire selon la revendication précédente, caractérisé par le fait que ledit acide est choisi parmi les acides acrylique, méthacrylique et maléique.

12. Nécessaire selon la revendication 10 ou 11, caractérisé par le fait que ledit dérivé est choisi parmi les anhydrides, les halogénures d'acide et les esters.

13. Nécessaire selon l'une quelconque des revendications 7 à 12, caractérisé par le fait que ledit autre monomère est choisi parmi la N-vinyl pyrrolidone, le méthylvinylether, l'éthylène, le propylène et le styrène.

14. Procédé de détection, avec amplification de signal, d'une séquence nucléotidique d'intérêt susceptible d'être immobilisée sur un support solide, dans lequel on utilise une sonde nucléotidique de détection marquée avec un traceur, caractérisé par le fait que l'on ajoute à un milieu liquide en contact avec ledit support solide, dans des conditions permettant l'hybridation :
- un réactif tel que défini dans l'une quelconque des revendications précédentes,
- et la sonde de détection marquée,
puis on révèle selon les méthodes usuelles la présence éventuelle du traceur immobilisé sur le support solide.

15. Procédé selon la revendication précédente, caractérisé par le fait que l'on ajoute le réactif et la sonde simultanément, c'est-à-dire sans étape de lavage intermédiaire.

16. Utilisation d'un réactif tel que défini dans l'une quelconque des revendications 1 à 13, comme réactif d'amplification de signal dans un procédé de détection d'une séquence nucléotidique d'intérêt.

## Patentansprüche

1. Kit zur Detektion einer interessierenden Nukleotidsequenz mit Signalamplifikation, umfassend mindestens eine mit einem Tracer markierte Nukleotidsonde, dadurch gekennzeichnet, daß der Kit in geeigneten Behältern ein in wäßrigem Milieu lösliches Reagens enthält, welches im wesentlichen ein Copolymer mit linearen im wesentlichen Kohlenstoffgrundgerüst umfaßt, das laterale Substituenten trägt, wobei die Kette aus einem ersten Typ von wiederkehrenden Einheiten und mindestens einem anderen Typ von wiederkehrenden Einheiten aufgebaut ist, bei denen mindestens ein Teil der Einheiten des ersten Typs einen eine Nukleotideinheit umfassenden lateralen Substituenten tragen, ein solcher lateraler Substituent auf den anderen Typen der Einheiten fehlt, jede der besagten Nukleotideinheiten, alle identisch, mindestens eine Nukleotidsequenz, die zur Hybridisierung mit der interessierenden Sequenz befähigt ist, und eine Nukleotidsequenz, die mit der Sonde hybridisieren kann, umfaßt,
wobei das Reagens im Mittel mehr als zwei Moläquivalente der Nukleotideinheiten pro Mol des Polymeren enthält.

2. Kit gemäß Anspruch 1, dadurch gekennzeichnet, daß in ein und derselben Nukleotideinheit die Sequenzen, die hybridisieren können, voneinander verschieden und nicht überlappend sind und daß die Nukleotidsequenz, die mit der Sonde hybridisieren kann, weiterhin eine willkürlich gewählte Sequenz ist.

3. Kit gemäß Anspruch 1, dadurch gekennzeichnet, daß in ein und derselben Nukleotidsequenz eine der besagten Sequenzen, die hybridisieren kann, in der anderen eingeschlossen ist.

4. Kit gemäß Anspruch 3, dadurch gekennzeichnet, daß in ein und derselben Nukleotidsequenz die zur Hybridisierung befähigten Sequenzen vereint sind und daß die Sonde in diesem Fall eine Sequenz enthält, die mit der Komplementärsequenz zu der interessierenden Sequenz hybridisieren kann.

5. Kit gemäß einem der vorstehenden Ansprüche, dadurch gekennzeichnet, daß das Reagens im Mittel 3 bis 100 Moläquivalente und insbesondere 5 bis 50 Moläquivalente der Nukleotideinheiten pro Mol des Copolymeren enthält.

6. Kit gemäß einem der vorstehenden Ansprüche, dadurch gekennzeichnet, daß das Copolymer ein Bipolymer ist.

7. Kit gemäß einem der vorstehenden Ansprüche, dadurch gekennzeichnet, daß die wiederkehrenden Einheiten aus der Copolymerisation eines ersten ungesättigten Monomeren mit mindestens einem anderen ungesättigten Monomeren stammen.

8. Kit gemäß dem vorstehenden Anspruch, dadurch gekennzeichnet, daß das erste ungesättigte Monomer eine reaktive Funktion trägt, die entweder direkt oder über die Vermittlung eines bifunktionellen Kupplungsmittels mit einem Vorläufer der besagten Nukleotideinheit zur Bildung des lateralen Substituenten reagieren kann.

9. Kit gemäß dem vorstehenden Anspruch, dadurch gekennzeichnet, daß die reaktive Funktion ausgewählt ist unter Amin-, Imid-, Aldehyd-, Epoxy-, Thiol-, Halogenalkyl- oder Carbonsäurefunktionen, wobei die Carbonsäurefunktion gegebenenfalls in Form der Säurehalogenide, des Anhydrids oder eines Esters aktiviert ist.

10. Kit gemäß dem vorstehenden Anspruch, dadurch gekennzeichnet, daß das erste Monomer ein ungesättigtes Carbonsäurederivat ist.

11. Kit gemäß dem vorstehenden Anspruch, dadurch gekennzeichnet, daß die Säure ausgewählt ist unter Acrylsäure, Methacrylsäure und Maleinsäure.

12. Kit gemäß Anspruch 10 oder 11, dadurch gekennzeichnet, daß das Derivat ausgewählt ist unter den Anhydriden, den Säurehalogeniden und den Estern.

13. Kit gemäß einem der Ansprüche 7 bis 12, dadurch gekennzeichnet, daß das andere Monomer ausgewählt ist unter N-Vinylpyrrolidon, Methylvinylether, Ethylen, Propylen und Styrol.

14. Verfahren zur Detektion mit Signalamplifikation einer interessierenden Nukleotidsequenz, die auf einem festen Träger immobilisiert werden kann, bei dem man eine mit einem Tracer markierte nukleotidische Nachweissonde einsetzt, dadurch gekennzeichnet, daß man unter die Hybridisierung gestattenden Bedingungen zu einem flüssigen Medium in Kontakt mit dem festen Träger zufügt:
- ein Reagens, definiert gemäß einem vorstehenden Ansprüche
- und die markierte Nachweissonde,
wonach man gemäß bekannter Verfahren die mögliche Anwesenheit des auf dem festen Träger immobilisierten Tracers ermittelt.

15. Verfahren gemäß dem vorstehenden Anspruch, dadurch gekennzeichnet, daß man das Reagens und die Sonde gleichzeitig, d. h. ohne zwischengeschalteten Waschschritt, zugibt.

16. Verwendung eines Reagens, definiert gemäß einem der Ansprüche 1 bis 13, als Signalamplifikationsreagens in einem Verfahren zur Detektion einer interessierenden Nukleotidsequenz.

## Claims

1. Kit for detecting a nucleotide sequence of interest, with signal amplification, which kit comprises at least one nucleotide probe which is labelled with a tracer, characterized in that it contains, in appropriate containers, a reagent which is soluble in aqueous medium and which essentially comprises a copolymer having an essentially carbonaceous linear skeleton carrying lateral substituents, whose chain consists of a first type of repetitive motifs and of at least one other type of repetitive motifs, in which at least a proportion of the motifs of the first type carry a lateral substituent comprising a nucleotide unit, with such a lateral substituent being absent from the other types of motifs, with each of the said nucleotide units, all of which are identical, at least comprising a nucleotide sequence which is able to hybridize with the said sequence of interest and a nucleotide sequence which is able to hybridize with the said probe,
and with the said reagent on average containing more than two of the said nucleotide units, in molar equivalents, per mole of polymer.

2. Kit according to Claim 1, characterized in that, in one and the same nucleotide unit, the said sequences able to hybridize are distinct and nonoverlapping, and in that the said nucleotide sequence which is able to hybridize with the probe is then a sequence which is chosen arbitrarily.

3. Kit according to Claim 1, characterized in that, in one and the same nucleotide unit, one of the said sequences which are able to hybridize is included in the other.

4. Kit according to Claim 3, characterized in that, in one and the same nucleotide unit, the said sequences which are able to hybridize are mixed up, and the probe then contains a sequence which is able to hybridize with the sequence which is complementary to the said sequence of interest.

5. Kit according to any one of the preceding claims, characterized in that the said reagent on average contains from 3 to 100, in particular from 5 to 50, of the said nucleotide units, in molar equivalents per mole of copolymer.

6. Kit according to any one of the preceding claims, characterized in that the said copolymer is a bipolymer.

7. Kit according to any one of the preceding claims, characterized in that the said repetitive motifs result from the copolymerization of a first unsaturated monomer with at least one other unsaturated monomer.

8. Kit according to the preceding claim, characterized in that the said first unsaturated monomer carries a reactive function which is able to react, either directly or by way of a bifunctional coupling agent, with a precursor of the said nucleotide unit in order to form the said lateral substituent.

9. Kit according to the preceding claim, characterized in that the said reactive function is selected from the amine, imide, aldehyde, epoxy, thiol, halogenoalkyl or carboxylic acid functions, with the carboxylic acid where appropriate being activated in the form of acid halide, in the form of anhydride or in the form of ester.

10. Kit according to the preceding claim, characterized in that the said first monomer is an unsaturated carboxylic acid derivative.

11. Kit according to the preceding claim, characterized in that the said acid is selected from acrylic, methacrylic and maleic acids.

12. Kit according to Claim 10 or 11, characterized in that the said derivative is selected from anhydrides, acid halides and esters.

13. Kit according to any one of Claims 7 to 12, characterized in that the said other monomer is selected from N-vinylpyrrolidone, methyl vinyl ether, ethylene, propylene and styrene.

14. Process for detecting, with signal amplification, a nucleotide sequence of interest which is capable of being immobilized on a solid support, in which process a tracer-labelled nucleotide detection probe is used, characterized in that:
- a reagent as defined in any one of the preceding claims,
- and the labelled detection probe,
are added to a liquid medium which is in contact with the said solid support, under conditions permitting hybridization, and conventional methods are then used to reveal the possible presence of the tracer immobilized on the solid support.

15. Process according to the preceding claim, characterized in that the reagent and the probe are added simultaneously, that is without any intermediate washing step.

16. Use of a reagent as defined in any one of Claims 1 to 13 as a signal-amplifying reagent in a process for detecting a nucleotide sequence of interest.
